(19) Europäisches Patentamt
European Patent Office
Office européen des brevets

(11) EP 4 171 483 B1

(12) EUROPEAN PATENT SPECIFICATION

(45) Date of publication and mention of the grant of the patent:
**08.04.2026 Bulletin 2026/15**

(21) Application number: **21742994.3**

(22) Date of filing: **24.06.2021**

(51) International Patent Classification (IPC):
***A61K 8/36*** (2006.01) ***A61K 8/365*** (2006.01)
***A61K 8/44*** (2006.01) ***A61K 8/46*** (2006.01)
***A61K 8/73*** (2006.01) ***A61K 8/81*** (2006.01)
***A61Q 5/02*** (2006.01) ***A61Q 19/10*** (2006.01)
***A61K 8/02*** (2006.01)

(52) Cooperative Patent Classification (CPC):
**A61K 8/44; A61K 8/027; A61K 8/36; A61K 8/365; A61K 8/466; A61K 8/731; A61K 8/732; A61K 8/8129; A61Q 5/02; A61Q 19/10**

(86) International application number:
**PCT/US2021/038786**

(87) International publication number:
**WO 2021/262912 (30.12.2021 Gazette 2021/52)**

(54) **DISSOLVABLE SOLID FIBROUS ARTICLES CONTAINING ANIONIC SURFACTANTS**

LÖSLICHE FESTE FASERARTIKEL MIT ANIONISCHEN TENSIDEN

ARTICLES FIBREUX, SOLIDES, SOLUBLES, CONTENANT DES TENSIOACTIFS ANIONIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.06.2020 US 202063044998 P**
**17.12.2020 US 202063126781 P**

(43) Date of publication of application:
**03.05.2023 Bulletin 2023/18**

(73) Proprietor: **The Procter & Gamble Company**
**Cincinnati, OH 45202 (US)**

(72) Inventor: **SONG, Brian, Xiaoqing**
**Cincinnati, Ohio 45202 (US)**

(74) Representative: **P&G Patent Germany**
**Procter & Gamble Service GmbH**
**Sulzbacher Straße 40**
**65824 Schwalbach am Taunus (DE)**

(56) References cited:
**WO-A2-2018/140676 US-A1- 2014 329 428**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 171 483 B1

**Description**

FIELD OF THE INVENTION

The present invention relates to fibrous articles, more particularly to dissolvable fibrous articles comprising one or more anionic surfactants, in particular anionic surfactants that are substantially free of or free of sulfate-based surfactants.

BACKGROUND OF THE INVENTION

Many personal care and other consumer products, including shampoos, in the market today are sold in liquid form. While widely used, liquid products often have tradeoffs in terms of packaging, storage, transportation, and convenience of use. For example, these products are generally formulated with a substantial amount of a liquid, such as water (e.g. ~80% or more), preservatives, and stabilizers, that add significant bulk and translates to inefficient, costly shipping and storage. Also, liquid personal care products can be difficult to use in terms of controlling dosage and the delivery of the product.

In order to overcome some of these drawbacks, it can be desirable to formulate personal care products as solid articles, such as fibrous articles. Fibrous articles can be desirable because the articles can quickly dissolve into a smooth composition. Fibrous articles that are shampoos can provide consumer acceptable amount of lather. For example patent applications WO 2018/140676 A2 and US 2014/329428 A1 disclose solid fibrous personal care articles including cleansing articles such as shampoo or body wash.

A fibrous article can be made from a filament-forming composition, which can be a melt that includes at least a polymeric structurant, a surfactant, and a volatile liquid solvent, such as water. The filament-forming composition can be spun via a spinning die into one or more fibrous elements. After spinning, the fibrous elements can be dried to remove the liquid solvent and then the fibrous elements can be collected on a belt to form a fibrous article comprising the fibrous elements.

It can be difficult to manufacture fibrous elements efficiently so these products can be sold in mass. One way to make fibrous articles more efficiently is to increase the level of surfactant in the melt from 30-35% to at least 40%, thereby decreasing the amount of liquid solvent that needs to be removed from the fibrous elements during the drying step. Efficiency could also be improved by increasing the melt temperature during spinning from 25°C to greater than 35°C to aid in evaporation. However, increasing the level of surfactant and/or raising the temperature can cause phase separation and/or breakdown of the rheology and elastic modulus, which can make it difficult to spin fibers.

Furthermore, to spin the melt composition into consumer acceptable fibrous elements, the melt composition needs to be viscous and elastic. However, when the final article that is made from this melt composition is rehydrated for use, it does not feel like a smooth, liquid shampoo, instead the dissolved articles returns to a melt-like gel that can feel sticky, stringy, and/or gooey in a user's hands.

As such, there remains a need for a melt composition that is phase stable and maintains its rheology (G') when the melt composition has at least 40% solids and a temperature greater than 35°C. There is also a need for a fibrous article that when it is hydrated, it forms a shampoo composition that is smooth and creamy.

SUMMARY OF THE INVENTION

A dissolvable solid fibrous shampoo article comprising a plurality of fibrous elements comprising: (a) from 1% to 50%, preferably from 20% to 70%, more preferably from 35% to 60%, by weight on a dry article basis, of a polymeric structurant, wherein the polymeric structurant is selected from carboxymethyl cellulose, starch, polyvinyl alcohol, or combinations thereof, wherein the total amount of polymeric structurant ranges from 1% to 50% by weight on a dry article basis; (b) from 20% to 70%, preferably from 40% to 70%, more preferably from 45% to 65%,by weight on a dry article basis, of a surfactant system comprising: (i) from 35% to 90%, preferably from 45% to 85%, more preferably from 50% to 80%, by weight of the surfactant system on a dry article basis, of a primary anionic surfactant, wherein the primary anionic surfactant comprises a glutamate surfactant selected from the group consisting of sodium cocoyl glutamate, disodium cocoyl glutamate, potassium cocoyl glutamate, dipotassium cocoyl glutamate, ammonium cocoyl glutamate, diammonium cocoyl glutamate, TEA-cocoyl glutamate, or combinations thereof or an alaninate surfactant selected from sodium cocoyl alaninate, sodium lauroyl alaninate, sodium N-dodecanoyl-1-alaninate, and combinations thereof, wherein the total amount of primary anionic surfactant ranges from 35% to 90% by weight of a dry article basis; and (ii) from 10% to 65%, preferably from 15% to 55%, more preferably from 23% to 50%, by weight of the surfactant system on a dry article basis, of a co-surfactant;wherein the surfactant system comprises less than 0.1% of sulfate-based surfactants by total weight of the fibrous elements; (c) from 0.5% to 5%, preferably from 0.75% to 3.7%, more preferably from 1% to 3%,by weight on a dry article basis, of a pH adjuster, wherein the pH adjuster consists of a monoprotic organic acid; and wherein the monoprotic organic acid is dispersed throughout the fibrous elements, wherein the monoprotic organic acid is selected from the group

consisting of lactic acid, acetic acid, glycolic acid, glyceric acid, and combinations thereof, wherein the total amount of pH adjuster ranges from 0.5% to 5% by weight of a dry article basis; (d) wherein the fibrous elements comprises less than 0.1% of citric acid by total weight of the fibrous elements; (e) wherein the plurality of fibrous elements are intertangled or otherwise associated with one another to form the fibrous article.

A melt composition suitable for obtaining the above article comprising: (a) from about 1% to about 50%, by weight, of a polymeric structurant; (b) from about 20% to about 70%, by weight, of a surfactant system comprising: (i) from about 35% to about 90%, by weight of the surfactant system, of a primary anionic surfactant; and (ii) from about from about 10% to about 65%, by weight of the surfactant system, of a co-surfactant; wherein the surfactant system is substantially free of sulfate-based surfactants; (c) a total % melt solids content of ≥ 40%; (d) a pH of from about 5.8 to about 7 according to the pH Test Method; (e) a G' ≥ 25 Pa at 40°C according to the Rheology Test Method; and wherein the melt composition is phase stable at 25°C and 40°C.

BRIEF DESCRIPTION OF THE DRAWINGS

While the specification concludes with claims particularly pointing out and distinctly claiming the subject matter of the present invention, it is believed that the invention can be more readily understood from the following description taken in connection with the accompanying drawings, in which:

FIG. 1A is a photograph of a glass slide with a melt composition with poor rheology for spinning fibrous elements;
FIGS. 1B and 1C are photographs of the melt composition with poor rheology for spinning fibrous elements, as demonstrated by trying to make a fibrous element by pulling apart two fingers;
FIG. 2A is a photograph of a glass slide with a melt composition with sufficient rheology for spinning fibrous elements;
FIG. 2B is a photograph of the melt composition with sufficient rheology for spinning fibrous elements, as demonstrated by trying to make a fibrous element by pulling apart two fingers;
FIG. 3 is an example of a fibrous article containing filaments;
FIG. 4 is a schematic representation of an example of a fibrous element;
FIG. 5 is a schematic representation of an example of a process for making fibrous elements of the present invention; and
FIG. 6 is a schematic representation of an example of a die with a magnified view used in the process of FIG. 5.

DETAILED DESCRIPTION OF THE INVENTION

It can be desirable to formulate personal care products, such as shampoos, as solid articles including solid fibrous articles. A benefit of fibrous articles is that they can rapidly dissolve into a liquid personal care article. However, for these articles to be commercially viable, the manufacturing process needs to be efficient and the article needs to dissolve into a homogeneous, smooth, creamy shampoo composition.

In order to improve manufacturing efficiency, it can be desirable to increase the level of surfactant in the melt from 30-35% to at least 40% and to increase the melt temperature during spinning from 25°C to greater than 35°C.

A fibrous article can be made from a filament-forming composition, which can be a melt that includes at least a polymeric structurant, a surfactant, and a volatile solvent, such as water. The filament-forming composition can be spun via a spinning die into one or more fibrous elements. Before the filament-forming composition is spun into fibrous elements, the pH is adjusted, typically with citric acid. The pH can be adjusted to a pH of about 6 so it is safe and effective on a person's hair and scalp. Furthermore, shampoos that include sulfate-free anionic surfactants, such as glutamate surfactants and alaninate surfactants, can perform better at a pH of about 6.

However, it was found that when the melt composition had ≥ 40% solids and heated to 40°C, when the citric acid was added, the melt composition coagulated, making it impossible to spin acceptable fibrous elements. Thus, a new pH adjusting agent needed to be identified that did not cause the melt to phase separate and maintained the melt's rheology (G' ≥ 50 Pa) in a melt that contains ≥ 40% solids and is heated to ≥ 35°C.

FIGS. 1A, 1B, and 1C are photographs that highlight a melt composition that does not have the proper rheology to form fibrous elements. The melt composition in FIGS. 1A, 1B, and 1C is 99.78% Example A from Table 1, below, with the addition of 0.22% citric acid (as shown in Table 4, below). This melt composition has a pH of 5.5. FIG. 1A is a photograph of a glass slide with the melt composition. The slide was made by trying to pull fibers out of a sample of the melt using a pipette tip and circling around the glass slide. However, instead of forming spinnable fibers, the glass slide shows globs of melt composition. Similarly, FIGS. 1B and 1C are photographs where the melt composition was put on a forefinger and to see if thin fibers could be made by putting the thumb and forefinger together and then pulling them apart. In both FIGS. 1B and 1C, the melt composition remains as a glob on the finger, instead of forming a thin fiber.

It was found that when lactic acid was added to the melt composition to adjust the pH to a melt containing ≥ 40% solids and is heated to ≥ 35°C, the melt maintained its rheology and phase stability.

FIGS. 2A and 2B are photographs that highlight a melt composition that has the proper rheology to form fibrous elements. The melt composition in FIGS. 2A and 2B are Example 1 from Table 1, below. Example 1 contains greater than 40% total melt solids content and includes lactic acid. The slide was made, as discussed above, with respect to FIG. 1A. However, unlike FIG. 1A, the slide shows fine fibrous elements that indication that this melt composition may be spinnable into fibrous elements. Furthermore, when the melt composition of Example 1 was placed between the thumb and forefinger, as shown in FIG. 2B, fine fibrous elements are formed when the fingers are moved apart.

However, fibrous articles made from this melt can still feel sticky, stringy and/or gooey after it is rehydrated in a user's hands instead of feeling smooth and creamy, like liquid shampoo. One way to prevent the stringiness of the rehydrated fibrous article would be to reduce the rheology of the melt. However, it can be very difficult to spin fibers from a melt that does not have a G' ≥ 50 Pa at 40°C. Therefore, an active agent can be added to the dried fibrous elements and/or article to reduce the elastic modulus when the product is wetted during use. The active agent can interact with the hydrated article resulting in a homogenous, non-sticky gel or cream when rubbed in between hands before applying to hair. The reduced stickiness also makes the product spread easier and more evenly onto hair fibers.

An additive, including but not limited to organic acids formed from diprotic and triprotic acids (e.g. citric acid) and salts thereof (e.g. sodium citrate) and/or a hydratrope (e.g. sodium xylene sulfonate), can be added as a coating to all or some of the fibrous elements and/or all or a portion of the fibrous article. The additive can be added to the solid fibrous structure and/or fibrous elements as a solid powder. In some examples, the additive can be added to the melt and can be distributed throughout the fibrous structure. The fibrous article and/or fibrous elements can contain from about 0.05% to about 5% additive, by weight of the article, alternatively from about 0.1% to about 3%, alternatively from about 0.2% to about 2%, alternatively from about 0.3% to about 1%, and alternatively from about 0.4% to about 0.7%. The additive can reduce the rheology/elasticity of the hydrated product, allowing the product to dissolve into a smooth, homogeneous liquid with reduced stickiness that is easy to spread evenly across a user's hair.

The additive can be an organic acid and salts thereof formed from diprotic and triprotic acids that are a solid at 25°C. Non-limiting examples of organic acids can include citric acid, oxalic acid, malonic acid, tartronic acid, fumaric acid, maleic acid, malic acid, tartaric acid, and salts thereof and combinations thereof. Non-limiting examples of salts thereof can include sodium citrate, sodium oxalate, sodium malonate, sodium tartrate, sodium maleate, and combinations thereof.

For example, when citric acid and was added to the outer surface of the article as a coating, it was found that citric acid reduced the rheology/elasticity by modifying the pH of the hydrated product (*see* Table 4 and Table 5, hereinafter) can be added to an article containing sulfate-free anionic surfactants. In another example, sodium citrate can be added to the outer surface of the article as a coating to reduce the rheology/elasticity of the hydrated /dissolved product (*see* Table 4, hereinafter). The citric acid can also form sodium citrate. In Table 5, the article contains a coating with citric acid and sodium bicarbonate. In this example, when hydrated a portion of the citric acid reacts with sodium bicarbonate to form sodium citrate and both the citric acid and sodium citrate work to change the rheology of the hydrated shampoo composition.

The additive can also be a hydratrope. Non-limiting examples of hydratropes can include sodium xylene sulfonate, urea, sodium toluenesulfonate, and combinations thereof. For example, sodium xylene sulfonate, can be added to the outer surface of the article as a coating.

The melt composition can have a total % melt solids content of ≥ 35%, alternatively ≥ 38%, alternatively ≥ 40%, alternatively ≥ 42%, and alternatively ≥ 45%. The melt composition can have a total % melt solids content from about 35% to about 60%, alternatively from about 38% to about 55%, and alternatively from about 40% to about 50%. The total % solids content is what would remain in the melt after the liquid evaporates and can include surfactant solids, polymers solids and salts from the raw materials.

The melt composition can have a pH of from about 5.5 to about 7.5, alternatively from about 5.8 to about 7, alternatively from about 6 to about 6.5, and alternatively from about 6.0 to about 6.3. The pH is determined using the pH Test Method, described hereafter.

The melt composition can have a G' at 40°C of ≥ 25 Pa, alternatively ≥ 30, alternatively ≥ 35, alternatively ≥ 40 Pa, alternatively ≥ 45 and alternatively ≥ 50 Pa. The melt composition can have a G' at 40°C of from 25 Pa to about 100 Pa, alternatively from about 30 Pa to about 95 Pa, alternatively from about 40 Pa to about 90 Pa, alternatively from about 45 Pa to about 80 Pa, alternatively from about 50 Pa to about 75 Pa. The G' can be determined by the Rheology Method, described hereafter. The G" at 40°C can be from about 30 Pa to about 300 Pa, alternatively from about 50 Pa to about 200 Pa, alternatively from about 70 Pa to about 160 Pa, and alternatively from about 80 Pa to about 150 Pa. The melt composition can have a tan delta (ratio of G"/G') at 40°C of from about 2.8 and 4.8, alternatively from about 2.9 to about 4.5, and alternatively from about 3 to about 4.2.

The melt composition and/or the hydrated fibrous element (as hydrated with 7 mL of warm tap water, from Mason, Ohio, per 2.5 g article and mixed until it formed a homogeneous shampoo product) at 25°C can have a G' (Pa), G" (Pa), and shear stress (Pa.s), as determined by the Rheology Test Method, described hereafter. The G' can be ≤ 200 Pa, alternatively ≤ 150 Pa, alternatively ≤ 100 Pa, alternatively ≤ 75 Pa, alternatively ≤ 60 Pa, and alternatively ≤ 50 Pa. The G' can be from 5 Pa to about 150 Pa, alternatively from about 10 Pa to about 100 Pa, alternatively from about 12 Pa to about 75 Pa, and alternatively from about 15 Pa to about 50 Pa. The G" can be ≤ 200 Pa, alternatively ≤ 150 Pa, alternatively

≤ 135 Pa, and alternatively ≤ 100 Pa. The G" can be from about 10 Pa to about 300 Pa, alternatively from about 20 Pa to about 200 Pa, alternatively from about 50 Pa to about 160 Pa, and alternatively from about 80 Pa to about 150 Pa. The shear stress can be ≤ 300 Pa.s, alternatively ≤ 200 Pa.s, alternatively ≤ 155 Pa.s, alternatively ≤ 100 Pa.s, alternatively ≤ 50 Pa.s, and alternatively ≤ 40 Pa.s. The shear stress can be from about 5 to about 300 Pa.s, alternatively from about 10 to about 200 Pa.s, alternatively from about 12 to about 150 Pa.s, alternatively from about 15 to about 100 Pa.s, and alternatively from about 25 to about 50 Pa.s.

The melt and/or fibrous element can contain a pH adjuster and the pH adjuster can be a monoprotic organic acid. The monoprotic organic acid can be selected from the group consisting of lactic acid, acetic acid, glycolic acid, glyceric acid, and combinations thereof. The monoprotic acid can be dispersed throughout the fibrous element. The pH adjuster can be present throughout the fibrous element. In some examples, the fibrous elements and/or fibrous articles do not comprise a coating. In other examples, the fibrous elements and/or fibrous article can comprise a coating and the coating can be substantially free of or free of a monoprotic acid.

The fibrous article and/or fibrous elements can contain from about 0.25% to about 5% pH adjuster, by weight of the melt and/or fibrous elements and/or fibrous article, alternatively from about 0.5% to about 4%, alternatively from about 0.6% to about 3.8%, alternatively from about 0.75% to about 3.7%, alternatively from about 0.8% to about 3.5%, alternatively from about 1% to about 3%, alternatively from about 1.25% to about 2%.

The melt and/or fibrous element and/or fibrous article can be substantially free of or free of diprotic and/or triprotic acids. In some examples, the fibrous elements and/or fibrous article can have a coating that comprises a diprotic and/or triprotic acid, while the fibrous elements can be substantially free of or free of diprotic and/or triprotic acid dispersed throughout the fibrous element. The diprotic acid can be selected from the group consisting of dicarboxylic acids including oxylic acid, malonic acid, tartronic acid, fumaric acid, maleic acid, malic acid, tartaric acid, and combinations thereof. The triprotic acid can be selected from the group consisting of phosphoric acid, citric acid, and combinations thereof. The melt and/or fibrous elements and/or article can be substantially free of or free of citric acid. The citric acid may not be present throughout the fibrous elements. The citric acid can be present in a coating on the fibrous article and/or fibrous elements. The dissolvable solid fibrous shampoo article comprises from 0.5% to 5%, preferably from 0.75% to 3.7%, more preferably from 1% to 3%,by weight on a dry article basis, of a pH adjuster, wherein the pH adjuster consists of a monoprotic organic acid; and wherein the monoprotic organic acid is dispersed throughout the fibrous elements, wherein the monoprotic organic acid is selected from the group consisting of lactic acid, acetic acid, glycolic acid, glyceric acid, and combinations thereof, wherein the total amount of pH adjuster ranges from 0.5% to 5% by weight of a dry article basis. The fibrous elements comprise less than 0.1% of citric acid by total weight of the fibrous elements

The melt and/or the fibrous elements and/or the fibrous article can have a weight ratio of total surfactant to total structurant ≥ 1.85, alternatively ≥ 1.9, alternatively ≥ 2.0, alternatively ≥ 2.2, alternatively ≥ 2.3, and alternatively ≥ 2.4. The melt and/or the fibrous elements and/or the fibrous article can have a weight ratio of total surfactant to total structurant from about 1.85 to about 4, alternatively from about 1.9 to about 3.75, alternatively from about 2 to about 3.5, alternatively from about 2.1 to about 3, alternatively from about 2.25 to about 2.75, and alternatively from about 2.3 to about 2.5.

The article can have a hand dissolution value, as determined by the Hand Dissolution Method, described hereafter, of less than about 25 strokes, alternatively less than about 15 strokes, alternatively less than 12 strokes, alternatively less than 10 strokes, alternatively from about 1 to about 25 strokes, alternatively from about 2 to about 15 strokes, alternatively from about 3 to about 10 strokes, alternatively from about 3 to about 9 strokes, and alternatively from about 3 to about 7 strokes.

The article can have from about 0.5% to about 50%, by weight of the article, of a coating, alternatively from about 1% to about 25%, alternatively from about 1.5% to about 15%, alternatively from about 2% to about 10%, alternatively from about 3% to about 8%, and alternatively from about 4% to about 7.5%.

<u>Definitions</u>

"Dissolvable" means that the Dissolvable Solid article is completely soluble in water or it provides a uniform dispersion upon mixing in water according to the Hand Dissolution Test, described hereafter. The Dissolvable Solid article can have a hand dissolution value of from about 1 to about 30 strokes, alternatively from about 2 to about 25 strokes, alternatively from about 3 to about 20 strokes, and alternatively from about 4 to about 15 strokes, as measured by the Hand Dissolution Method.

"Fibrous article" as used herein means a structure that comprises one or more fibrous elements and optionally, one or more particles and/or coatings. In one example, a fibrous article according to the present invention means an association of fibrous elements and optionally, particles and/or coatings that together form a structure, such as a unitary structure, capable of performing a function.

FIG. 3 is an example of a fibrous article containing filaments.

The fibrous articles of the present invention may be homogeneous or may be layered. If layered, the fibrous articles may comprise at least two and/or at least three and/or at least four and/or at least five layers, for example one or

more fibrous element layers, one or more particle layers and/or one or more fibrous element/particle mixture layers. A layer may comprise a particle layer within the fibrous article or between fibrous element layers within a fibrous article. A layer comprising fibrous elements may sometimes be referred to as a ply. A ply may be a fibrous article which may be homogeneous or layered as described herein.

In one example, a single-ply fibrous article according to the present invention or a multi-ply fibrous article comprising one or more fibrous article plies according to the present invention may exhibit a basis weight of less than 5000 g/m$^2$ as measured according to the Basis Weight Test Method described herein. In one example, the single- or multi-ply fibrous article according to the present invention may exhibit a basis weight of greater than 10 g/m$^2$ to about 5000 g/m$^2$ and/or greater than 10 g/m$^2$ to about 3000 g/m$^2$ and/or greater than 10 g/m$^2$ to about 2000 g/m$^2$ and/or greater than 10 g/m$^2$ to about 1000 g/m$^2$ and/or greater than 20 g/m$^2$ to about 800 g/m$^2$ and/or greater than 30 g/m$^2$ to about 600 g/m$^2$ and/or greater than 50 g/m$^2$ to about 500 g/m$^2$ and/or greater than 100 g/m$^2$ to about 800 g/m$^2$ and/or greater than 200 g/m$^2$ to about 600 g/m$^2$ as measured according to the Basis Weight Test Method.

In one example, the fibrous article of the present invention can be a "unitary fibrous article." "Unitary fibrous article" as used herein is an arrangement comprising a plurality of two or more and/or three or more fibrous elements that are inter-entangled or otherwise associated with one another to form a fibrous article. The unitary fibrous article can optionally contain particles. A unitary fibrous article of the present invention may be one or more plies within a multi-ply fibrous article. In one example, a unitary fibrous article of the present invention may comprise three or more different fibrous elements. In another example, a unitary fibrous article of the present invention may comprise two different fibrous elements, for example a co-formed fibrous article, upon which a different fibrous element is deposited to form a fibrous article comprising three or more different fibrous elements.

"Article" as used herein refers to a consumer use unit, a consumer unit dose unit, a consumer use saleable unit, a single dose unit, or other use form comprising a unitary fibrous article and/or comprising one or more fibrous articles of the present invention.

"By weight on a dry filament basis" means that the weight of the filament measured immediately after the filament has been conditioned in a conditioned room at a temperature of 22 °C $\pm$ 2 °C and a relative humidity of 42% $\pm$ 4% for 2 hours. Similarly, "by weight on a dry fibrous element basis" or "by weight on a dry fibrous article basis" means the weight of the fibrous element or structure after the fibrous element has been conditioned in a conditioned room at a temperature of 22 °C $\pm$ 2 °C and a relative humidity of 42% $\pm$ 4% for 2 hours.

"Fibrous element" as used herein means an elongate particulate having a length greatly exceeding its average diameter, i.e. a length to average diameter ratio of at least about 10. A fibrous element may be a filament or a fiber. In one example, the fibrous element can be a single fibrous element rather than a yarn comprising a plurality of fibrous elements.

The fibrous elements of the present invention may be spun from a filament-forming composition also referred to as fibrous element-forming compositions via suitable spinning process operations, such as meltblowing, spunbonding, electro-spinning, and/or rotary spinning.

The fibrous elements of the present invention may be monocomponent and/or multicomponent. For example, the fibrous elements may comprise bicomponent fibers and/or filaments. The bicomponent fibers and/or filaments may be in any form, such as side-by-side, core and sheath, islands-in-the-sea and the like.

"Filament" as used herein means an elongate particulate as described above that exhibits a length of greater than or equal to 5.08 cm (2 in.) and/or greater than or equal to 7.62 cm (3 in.) and/or greater than or equal to 10.16 cm (4 in.) and/or greater than or equal to 15.24 cm (6 in.).

Filaments are typically considered continuous or substantially continuous in nature. Filaments are relatively longer than fibers. Non-limiting examples of filaments include meltblown and/or spunbond filaments. Non-limiting examples of polymers that can be spun into filaments include natural polymers, such as starch, starch derivatives, cellulose, such as rayon and/or lyocell, and cellulose derivatives, hemicellulose, hemicellulose derivatives, and synthetic polymers including, but not limited to thermoplastic polymer filaments, such as polyesters, nylons, polyolefins such as polypropylene filaments, polyethylene filaments, and biodegradable thermoplastic fibers such as polylactic acid filaments, polyhydroxyalkanoate filaments, polyesteramide filaments and polycaprolactone filaments.

"Fiber" as used herein means an elongate particulate as described above that exhibits a length of less than 5.08 cm (2 in.) and/or less than 3.81 cm (1.5 in.) and/or less than 2.54 cm (1 in.).

Fibers are typically considered discontinuous in nature. Non-limiting examples of fibers include staple fibers produced by spinning a filament or filament tow of the present invention and then cutting the filament or filament tow into segments of less than 5.08 cm (2 in.) thus producing fibers.

In one example, one or more fibers may be formed from a filament of the present invention, such as when the filaments are cut to shorter lengths (such as less than 5.08 cm in length). Thus, in one example, the present invention also includes a fiber made from a filament of the present invention, such as a fiber comprising one or more polymeric structurants and one or more other ingredients, such as surfactants and high melting point fatty materials. Therefore, references to filament and/or filaments of the present invention herein also include fibers made from such filament and/or filaments unless otherwise noted. Fibers are typically considered discontinuous in nature relative to filaments, which are

considered continuous in nature.

"Filament-forming composition" and/or "fibrous element-forming composition" as used herein means a composition that can be suitable for making a fibrous element of the present invention such as by meltblowing and/or spunbonding. The filament-forming composition comprises one or more polymeric structurants that exhibit properties that make them suitable for spinning into a fibrous element. In addition, the filament-forming composition may comprise one or more polar solvents, such as water, into which one or more, for example all, of the polymeric structurant and/or one or more, for example all, of surfactants are dissolved and/or dispersed prior to spinning a fibrous element, such as a filament from the filament-forming composition.In one example as shown in FIG. 4, a filament 10 of the present invention made from a filament-forming composition of the present invention is such that one or more actives 12, for example one or more active agents, may be present in the filament rather than on the filament, such as a coating composition comprising one or more active agents, which may be the same or different from the active agents in the fibrous elements and/or particles.

In one example, one or more additives, such as active agents, may be present in the fibrous element and one or more additional additives, such as active agents, may be present on a surface of the fibrous element. In another example, a fibrous element of the present invention may comprise one or more additives, such as active agents, that are present in the fibrous element when originally made, but then bloom to a surface of the fibrous element prior to and/or when exposed to conditions of intended use of the fibrous element.

As used herein, "vinyl pyrrolidone copolymer" (and "copolymer" when used in reference thereto) refers to a polymer of the following structure (I):

(I)

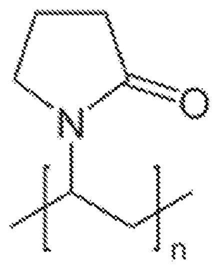

In structure (I), n is an integer such that the polymeric structurant has the degree of polymerization such that it possesses characteristics described herein. For purposes of clarity, the use of the term "copolymer" is intended to convey that the vinyl pyrrolidone monomer can be copolymerized with other non-limiting monomers such as vinyl acetate, alkylated vinyl pyrrolidone, vinyl caprolactam, vinyl valerolactam, vinyl imidazole, acrylic acid, methacrylate, acrylamide, methacrylamide, dimethacrylamide, alkylaminomethacrylate, and alkylaminomethacrylamide monomers.

As used herein, "vinyl acetate-vinyl alcohol copolymer" (and "copolymer" when used in reference thereto) refers to a polymer of the following structure (I):

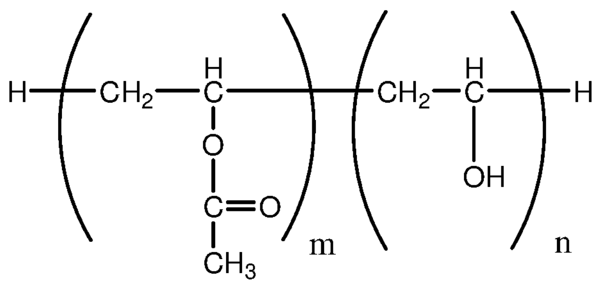

(I)

In structure (I), m and n are integers such that the polymeric structurant has the degree of polymerization and percent alcohol characteristics described herein. For purposes of clarity, this use of the term "copolymer" is intended to convey that the partially hydrolyzed polyvinyl acetate of the present invention comprises vinyl alcohol and vinyl acetate units. As discussed below, the polymeric structurant is routinely prepared by polymerizing vinyl acetate monomer followed by hydrolysis of some of the acetate groups to alcohol groups, as opposed to polymerization of vinyl acetate and vinyl alcohol monomer units (due in-part to the instability of vinyl alcohol).

"Conditions of intended use" as used herein means the temperature, physical, chemical, and/or mechanical conditions that a fibrous element and/or particle and/or fibrous article of the present invention is exposed to when the fibrous element and/or particle and/or fibrous article is used for one or more of its designed purposes. For instance, if a fibrous element and/or a particle and/or a fibrous article comprising a fibrous element is designed to be used by a human as a shampoo for hair care purposes, the conditions of intended use will include those temperature, chemical, physical and/or mechanical conditions present during the shampooing of the human's hair. Likewise, if a fibrous element and/or a particle and/or a fibrous article comprising a fibrous element is designed to be used in a dishwashing operation, by hand or by a dishwashing machine, the conditions of intended use will include the temperature, chemical, physical and/or mechanical conditions present in a dishwashing water and/or dishwashing machine, during the dishwashing operation.

"Active agent" as used herein means an additive that produces an intended effect in an environment external to a fibrous element and/or a particle and/or a fibrous article comprising a fibrous element of the present invention, such as when the fibrous element and/or a particle and/or fibrous article is exposed to conditions of intended use of the fibrous element and/or a particle and/or a fibrous article comprising a fibrous element. In one example, an active agent comprises an additive that treats a surface, including a soft surface (i.e., hair, skin). In another example, an active agent comprises an additive that creates a chemical reaction (i.e., foaming, fizzing, coloring, warming, cooling, lathering, disinfecting and/or clarifying and/or chlorinating, such as in clarifying water and/or disinfecting water and/or chlorinating water). In yet another example, an active agent comprises an additive that treats an environment (i.e., deodorizes, purifies, perfumes). In one example, the active agent is formed in situ, such as during the formation of the fibrous element and/or particle containing the active agent, for example the fibrous element and/or particle may comprise a dissolvable polymer (e.g., starch) and/or a surfactant (e.g., anionic surfactant), which may create a polymer complex or coacervate that functions as the active agent used to treat the hair and/or scalp. In one example, the active agent can include all compositions in the melt, fibrous elements, and/or fibrous article other than the polymeric structurant, including, but not limited to the surfactants and additives.

"Treats" as used herein with respect to treating a surface means that the active agent provides a benefit to a surface or environment. Treats includes regulating and/or immediately improving a surface's, cleanliness, smell, purity and/or feel. In one example treating in reference to treating a keratinous tissue (for example skin and/or hair) surface means regulating and/or immediately improving the keratinous tissue's cosmetic appearance and/or feel. For instance, "regulating skin, hair, or nail (keratinous tissue) condition" includes: thickening of skin, hair, or nails (e.g, building the epidermis and/or dermis and/or sub-dermal [e.g., subcutaneous fat or muscle] layers of the skin, and where applicable the keratinous layers of the nail and hair shaft) to reduce skin, hair, or nail atrophy, increasing the convolution of the dermal-epidermal border (also known as the rete ridges), preventing loss of skin or hair elasticity (loss, damage and/or inactivation of functional skin elastin) such as elastosis, sagging, loss of skin or hair recoil from deformation; melanin or non-melanin change in coloration to the skin, hair, or nails such as under eye circles, blotching (e.g., uneven red coloration due to, e.g., rosacea) (hereinafter referred to as "red blotchiness"), sallowness (pale color), discoloration caused by telangiectasia or spider vessels, and graying hair.

"Weight ratio" as used herein means the ratio between two materials on their dry basis.

"Water-soluble material" as used herein means a material that is miscible in water. **In** other words, a material that is capable of forming a stable (does not separate for greater than 5 minutes after forming the homogeneous solution) homogeneous solution with water at ambient conditions. "Water-insoluble" as used herein is meant that the material, particle, and/or substrate that does not dissolve in or readily break apart upon immersion in water. **In** some instances, water-insoluble materials swell when exposed to water.

"Ambient conditions" as used herein means 22 °C $\pm$ 2 °C and a relative humidity of 42% $\pm$ 4%.

As used herein, "molecular weight" or "M.Wt." refers to the weight average molecular weight unless otherwise stated. Molecular weight is measured using industry standard method, gel permeation chromatography ("GPC").

"Length" as used herein, with respect to a fibrous element, means the length along the longest axis of the fibrous element from one terminus to the other terminus. If a fibrous element has a kink, curl or curves in it, then the length is the length along the entire path of the fibrous element from one terminus to the other terminus.

"Diameter" as used herein, with respect to a fibrous element, is measured according to the Diameter Test Method described herein. In one example, a fibrous element of the present invention exhibits a diameter of less than 100 $\mu$m and/or less than 75 $\mu$m and/or less than 50 $\mu$m and/or less than 25 $\mu$m and/or less than 20 $\mu$m and/or less than 15 $\mu$m and/or less than 10 $\mu$m and/or less than 6 $\mu$m and/or greater than 1 $\mu$m and/or greater than 3 $\mu$m.

"Triggering condition" as used herein in one example means anything, as an act or event, that serves as a stimulus and initiates or precipitates a change in the fibrous element and/or particle and/or fibrous article of the present invention, such as a loss or altering of the fibrous element's and/or fibrous article's physical structure and/or a release of an additive, such as an active agent therefrom. In another example, the triggering condition may be present in an environment, such as water, when a fibrous element and/or particle and/or fibrous article of the present invention is added to the water. In other words, nothing changes in the water except for the fact that the fibrous element and/or fibrous article of the present invention is added to the water.

"Morphology changes" as used herein with respect to a fibrous element's and/or particle's morphology changing means that the fibrous element experiences a change in its physical structure. Non-limiting examples of morphology changes for a fibrous element and/or particle of the present invention include dissolution, melting, swelling, shrinking, breaking into pieces, exploding, lengthening, shortening, and combinations thereof. The fibrous elements and/or particles of the present invention may completely or substantially lose their fibrous element or particle physical structure or they may have their morphology changed or they may retain or substantially retain their fibrous element or particle physical structure as they are exposed to conditions of intended use.

"By weight on a dry fibrous element basis" and/or "by weight on a dry fibrous article basis" means the weight of the fibrous element and/or particle and/or fibrous article, respectively, measured immediately after the fibrous element and/or particle and/or fibrous article, respectively, has been conditioned in a conditioned room at a temperature of 22 °C ± 2 °C and a relative humidity of 42% ± 4% for 2 hours. In one example, by weight on a dry fibrous element basis and/or dry fibrous article basis means that the fibrous element and/or particle and/or fibrous article comprises less than 20% and/or less than 15% and/or less than 10% and/or less than 7% and/or less than 5% and/or less than 3% and/or to 0% and/or to greater than 0% based on the dry weight of the fibrous element and/or particle and/or fibrous article of moisture, such as water, for example free water, as measured according to the Water Content Test Method described herein.

"Total level" as used herein, for example with respect to the total level of one or more active agents present in the fibrous element and/or particle and/or fibrous article, means the sum of the weights or weight percent of all of the subject materials, for example active agents. In other words, a fibrous element and/or particle and/or fibrous article may comprise 25% by weight on a dry fibrous element basis and/or dry fibrous article basis of an anionic surfactant, 15% by weight on a dry fibrous element basis and/or dry fibrous article basis of a nonionic surfactant, 10% by weight of a chelant on a dry fibrous element basis and/or dry fibrous article basis, and 5% by weight of a perfume a dry fibrous element basis and/or dry fibrous article basis so that the total level of active agents present in the fibrous element and/or particle and/or fibrous article is greater than 50%; namely 55% by weight on a dry fibrous element basis and/or dry fibrous article basis.

"Fibrous article product" as used herein means a solid form, for example a rectangular solid, sometimes referred to as a sheet, that comprises one or more active agents, for example a hair care active agent, a shampoo active agent, a conditioning active agent, and mixtures thereof. In one example, a fibrous article product of the present invention comprises one or more surfactants, one or more enzymes (such as in the form of an enzyme prill), one or more perfumes and/or one or more suds suppressors. In another example, a fibrous article product of the present invention comprises a builder and/or a chelating agent. In another example, a fibrous article product of the present invention comprises a bleaching agent (such as an encapsulated bleaching agent).

"Associate," "Associated," "Association," and/or "Associating" as used herein with respect to fibrous elements and/or particle means combining, either in direct contact or in indirect contact, fibrous elements and/or particles such that a fibrous article is formed. In one example, the associated fibrous elements and/or particles may be bonded together for example by adhesives and/or thermal bonds. In another example, the fibrous elements and/or particles may be associated with one another by being deposited onto the same fibrous article making belt and/or patterned belt.

"Ply" or "Plies" as used herein means an individual fibrous article optionally to be disposed in a substantially contiguous, face-to-face relationship with other plies, forming a multiple ply fibrous article. It is also contemplated that a single fibrous article can effectively form two "plies" or multiple "plies", for example, by being folded on itself.

The term "free of" as used herein means that the melt composition, or the fibrous elements, or the fibrous article, comprises 0% of an ingredient by total weight of the composition, or by total weight of the fibrous article, or by total weight of the article, thus no detectable amount of the stated ingredient.

The term "substantially free of" as used herein means less than 1%, less than 0.8%, less than 0.5%, less than 0.3%, less than 0.1%, or less than an immaterial amount of a stated ingredient by total weight of the melt composition, or by total weigh of the fibrous elements, or by total weight of the fibrous article.

As used herein, the articles "a" and "an" when used herein, for example, "an anionic surfactant" or "a fiber" is understood to mean one or more of the material that is claimed or described.

As used herein, the terms "include," "includes," and "including," are meant to be non-limiting.

All percentages and ratios are calculated by weight unless otherwise indicated. All percentages and ratios are calculated based on the total composition unless otherwise indicated.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

Unless otherwise noted, all component or composition levels are in reference to the active level of that component or composition, and are exclusive of impurities, for example, residual solvents or by-products, which may be present in commercially available sources.

Fibrous article

The fibrous article can comprise a plurality of fibrous elements, for example a plurality of filaments. The fibrous article can include: fibrous elements containing a polymeric structurant, a surfactant system, and optionally an additive that can be in the fibrous elements or a coating.

FIG. 4 is a schematic representation of an example of a fibrous element according to the present invention. The filament can be homogenous.

In one example, the fibrous article comprises a plurality of identical or substantially identical from a compositional perspective of fibrous elements. In another example, the fibrous article may comprise two or more different fibrous elements according to the present invention. Non-limiting examples of differences in the fibrous elements may be physical differences such as differences in diameter, length, texture, shape, rigidness, elasticity, and the like; chemical differences such as crosslinking level, solubility, melting point, Tg, active agent, polymeric structurant, color, level of active agent, basis weight, level of polymeric structurant, presence of any coating on fibrous element, biodegradable or not, hydrophobic or not, contact angle, and the like; differences in whether the fibrous element loses its physical structure when the fibrous element is exposed to conditions of intended use; differences in whether the fibrous element's morphology changes when the fibrous element is exposed to conditions of intended use; and differences in rate at which the fibrous element releases one or more of its active agents when the fibrous element is exposed to conditions of intended use. In one example, two or more fibrous elements and/or particles within the fibrous article may comprise different active agents. This may be the case where the different active agents may be incompatible with one another, for example an anionic surfactant (such as a shampoo active agent) and a cationic surfactant (such as a hair conditioner active agent).

In another example, the fibrous article may exhibit different regions, such as different regions of basis weight, density and/or caliper. In yet another example, the fibrous article may comprise texture on one or more of its surfaces. A surface of the fibrous article may comprise a pattern, such as a non-random, repeating pattern. The fibrous article may be embossed with an emboss pattern. In another example, the fibrous article may comprise apertures. The apertures may be arranged in a non-random, repeating pattern.

In one example, the fibrous article of the present invention exhibits a thickness of greater than 0.01 mm and/or greater than 0.05 mm and/or greater than 0.1 mm and/or to about 100 mm and/or to about 50 mm and/or to about 20 mm and/or to about 10 mm and/or to about 5 mm and/or to about 2 mm and/or to about 0.5 mm and/or to about 0.3 mm as measured by the Thickness Test Method described herein.

For fibrous articles, the structure can comprise a significant number of dissolvable fibers with an average diameter less than about 150 micron, alternatively less than about 100 micron, alternatively less than about 10 micron, and alternatively less than about 1 micron with a relative standard deviation of less than 100%, alternatively less than 80%, alternatively less than 60%, alternatively less than 50%, such as in the range of 10% to 50%, for example. As set forth herein, the significant number means at least 10% of all the dissolvable fibers, alternatively at least 25% of all the dissolvable fibers, alternatively at least 50% of all the dissolvable fibers, alternatively at least 75% of all the dissolvable fibers. The significant number may be at least 99% of all the dissolvable fibers. Alternatively, from about 50% to about 100% of all the dissolvable fibers may have an average diameter less than about 10 micron. The dissolvable fibers produced by the method of the present disclosure can have a significant number of dissolvable fibers with an average diameter less than about 1 micron, or sub-micron fibers. In an embodiment, fibrous article may have from about 25% to about 100% of all the dissolvable fibers with an average diameter less than about 1 micron, alternatively from about 35% to about 100% of all the dissolvable fibers with an average diameter less than about 1 micron, alternatively from about 50% to about 100% of all the dissolvable fibers with an average diameter less than about 1 micron, and alternatively from about 75% to about 100% of all the dissolvable fibers with an average diameter less than about 1 micron.

The article can be characterized in one aspect by its Specific Surface Area. The aticle can have a Specific Surface Area of from about 0.03 $m^2/g$ to about 0.25 $m^2/g$, alternatively from about 0.035 $m^2/g$ to about 0.22 $m^2/g$, alternatively from about 0.04 $m^2/g$ to about 0.19 $m^2/g$, and alternatively from about 0.045 $m^2/g$ to about 0.16 $m^2/g$.

The structure can be a flat, flexible structure in the form of a pad, a strip, or tape and having a thickness of from about 0.5 mm to about 10 mm, alternatively from about 1 mm to about 9 mm, alternatively from about 2 mm to about 8 mm, and alternatively from about 3 mm to about 7 mm as measured by the below methodology. The Structure can be a sheet having a thickness from about 5mm to about 6.5mm. Alternatively, two or more sheets are combined to form a Structure with a thickness of about 5mm to about 10mm.

The structure can have a basis weight of from about 200 grams/$m^2$ to about 2,000 grams/$m^2$, alternatively from about 400 g/$m^2$ to about 1,200 g/$m^2$, alternatively from about 600 g/$m^2$ to about 2,000 g/$m^2$, and alternatively from about 700 g/$m^2$ to about 1,500 g/$m^2$.

The structure can have a dry density of from about 0.08 g/$cm^3$ to about 0.40 g/$cm^3$, alternatively from about 0.08 g/$cm^3$ to about 0.38 g/$cm^3$, alternatively from about 0.10 g/$cm^3$ to about 0.25 g/$cm^3$, and alternatively from about 0.12 g/$cm^3$ to about 0.20 g/$cm^3$.

Non-limiting examples of other fibrous articles suitable for the present invention are disclosed in U.S. Pat. Nos.

8,980,816 and 9,139,802, U.S. Pub. No. 2013/0171421, and U.S. App. No. 16/912,876.

Fibrous Elements

The fibrous element, such as a filament and/or fiber, of the present invention comprises one or more polymeric structurants, , wherein the polymeric structurant is selected from carboxymethyl cellulose, starch, polyvinyl alcohol, or combinations thereof. In addition to the polymeric structurants, the fibrous element may further comprise a surfactant system and optional ingredients including relatively high weight average molecular weight cationic polymers. Examples of fibrous elements can be found at U.S. Pat. App. 16/431,115.

Polymeric Structurant

The melt composition, and/or dissolvable fibrous article and/or fibrous elements can contain from about 1% to 90%, alternatively 10% to about 80%, alternatively from about 20% to about 70%, alternatively from about 30% to about 65%, alternatively from about 35% to about 60%, alternatively from about 20% to about 40%, of a polymeric structurant by weight on a dry fibrous element basis and/or a dry dissolvable fibrous article basis and/or by weight of the fibrous element-forming composition.

Non-limiting examples of fibrous-element forming polymeric structurant materials include water-soluble polymers. The water-soluble polymers may be synthetic or natural original and may be chemically and/or physically modified. The polar solvent-soluble polymers may exhibit a weight average molecular weight of from about 10,000 g/mol to about 40,000,000 g/mol, preferably from about 20,000 g/mol to about 30,000,000 g/mol, more preferably from about 35,000 g/mol to about 20,000,000 g/mol, even more preferably from about 40,000 g/mol to about 5,000,000 g/mol, most preferably from about 40,000 g/mol to about 500,000 g/mol.

The one or more fibrous-element forming polymeric structurants comprise one or more polyvinyl alcohols. The one or more polyvinyl alcohols may exhibit a weight average molecular weight of from about 10,000 g/mol to about 40,000,000 g/mol, alternatively from about 20,000 g/mol to about 30,000,000 g/mol, alternatively from about 35,000 g/mol to about 20,000,000 g/mol, alternatively from about 40,000 g/mol to about 5,000,000 g/mol, alternatively from about 40,000 g/mol to about 500,000 g/mol.

The one or more fibrous-element forming polymeric structurant materials may comprise two or more polyvinyl alcohols. One of the two or more polyvinyl alcohols may exhibit a weight average molecular weight of from about 10,000 g/mol to about 100,000 g/mol, alternatively from about 20,000 g/mol to about 50,000 g/mol, alternatively from about 25,000 g/mol to about 45,000 g/mol, and the other of two or more polyvinyl alcohols may exhibit a weight average molecular weight of from about 105,000 g/mol to about 40,000,000 g/mol, preferably from about 110,000 g/mol to about 20,000,000 g/mol, more preferably from about 120,000 g/mol to about 500,000 g/mol.

Non-limiting examples of fibrous-element forming polymeric structurant include water-soluble hydroxyl polymers, water-soluble thermoplastic polymers, water-soluble biodegradable polymers, water-soluble non-biodegradable polymers and mixtures thereof.

The one or more fibrous-element forming polymeric structurant materials may further comprise starch. Preferably, the one or more fibrous-element forming polymeric structurant materials may comprise one or more polyvinyl alcohols and starch.

The one or more fibrous-element forming materials may further comprise carboxymethyl cellulose. The one or more fibrous-element forming polymeric structurant materials may comprise one or more polyvinyl alcohols and carboxymethyl cellulose.

Surfactants

The melt composition and/or the dissolvable fibrous article and/or fibrous elements can contain from about 10% to about 90%, alternatively from about 20% to about 80%, alternatively from about 30% to about 75%, and alternatively from about 40% to about 70%, from about 45% to about 65%, of a surfactant system on by weight on a dry fibrous element basis and/or a dry dissolvable fibrous article basis and/or by weight of the fibrous element-forming composition.

Suitable anionic surfactants can include alkyl and alkyl ether sulfates. Other suitable anionic surfactants are the water-soluble salts of organic, sulfuric acid reaction products. Still other suitable anionic surfactants are the reaction products of fatty acids esterified with isethionic acid and neutralized with sodium hydroxide. Other similar anionic surfactants are described in U.S. Patent Nos. 2,486,921; 2,486,922; and 2,396,278.

Exemplary anionic surfactants include ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, potassium lauryl sulfate, potassium laureth

sulfate, sodium lauryl sarcosinate, sodium lauroyl sarcosinate, lauryl sarcosine, cocoyl sarcosine, ammonium cocoyl sulfate, ammonium lauroyl sulfate, sodium cocoyl sulfate, sodium lauroyl sulfate, potassium cocoyl sulfate, potassium lauryl sulfate, triethanolamine lauryl sulfate, triethanolamine lauryl sulfate, monoethanolamine cocoyl sulfate, monoethanolamine lauryl sulfate, sodium tridecyl benzene sulfonate, sodium dodecyl benzene sulfonate, sodium cocoyl isethionate and combinations thereof. In one embodiment, the anionic surfactant is sodium lauryl sulfate or sodium laureth sulfate.

In one embodiment, the anionic surfactant is at least one branched sulfate having the formula $CH_3-(CH_2)_z-CH(R^1)-CH_2-O-(CH_2CH(R^2)O)_y-SO_3M$; where z is from about 3 to about 14; $R^1$ represents H or a hydrocarbon radical comprising 1 to 4 carbon atoms, $R^2$ is H or $CH_3$; $R^1$ and $R^2$ are not both H; y is 0 to about 7; the average value of y is about 1 when y is not = 0; and M is a mono-valent or di-valent, positively-charged cation. Examples of mono-valent positively charged cations include ammonium, sodium, potassium, triethanolamine cation, and examples of di-valent positively charged cations include magnesium. For the foregoing branched sulfates, "average value" means that whereas the composition may comprise molecules having a value of y of other than 1, the average value of y all molecules in the composition is about 1.

The surfactant system is substantially free or free of sulfate-based surfactants including alkyl sulfate and alkyl ether sulfate type of surfactant. Alternatively, the dissolvable fibrous article does not comprise any alkyl sulfate which comprises $C_{10}$-$C_{18}$ alkyl sulfate or any alkyl ether sulfate including alkyl glyceryl ether sulfates.

In some examples, the dissolvable fibrous article may not comprise any alkyl ether sulfates which have the formula:

$$RO(CH_2CH_2O)_nSO_3M$$

wherein R is an alkyl or alkenyl having 8 to 18 carbons, alternatively 12 to 18 carbons, n has an average value of greater than at least 0.5, alternatively between 2 and 3; and M is a solubilizing cation such as sodium, potassium, ammonium or substituted ammonium.

In some examples, the dissolvable fibrous article may not comprise any ammonium and sodium lauryl ether sulfates.

If the dissolvable fibrous article does contain alkyl sulfate and/or alkyl ether sulfate type of surfactant, its content of such a weight proportion of: alkyl sulfates or alkyl ether sulfate type surfactant is less than or equal to the sum of 0.6, alternatively less than or equal to the sum of 0.2, alternatively equal to 0.

Also, the product may be substantially free of or free of alkyl sulfate and alkyl ether sulfate type of surfactant, as described hereinbefore.

The one or more active agents comprise one or more surfactants, wherein the one or more surfactants comprise at least one glutamate surfactant according to the general formula (I):

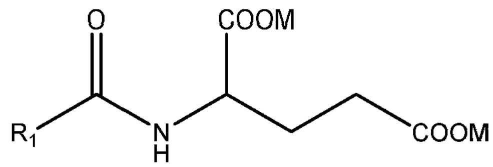
(I)

wherein $R_1$ can be saturated or unsaturated, straight or branched alkyl or alkenyl chain with from 5 to 20 carbon atoms, alternatively with from 7 to 17 carbon atoms, alternatively with from 9 to 13 carbon atoms; and M can be H, ammonium, triethanolammonium (TEA), sodium or potassium and mixtures thereof.

As set out above, the dissolvable fibrous article can be substantially free of or free of alkyl sulfate and alkyl ether sulfate type of surfactants.

The surfactant system can contain from an anionic primary surfactant. The article can contain from about 5% to about 70%, alternatively from about 10% to about 65%, alternatively from about 15% to about 55%, alternatively from about 20% to about 50% primary surfactant by weight of by weight on a dry fibrous element basis and/or a dry dissolvable fibrous article basis and/or by weight of the fibrous element-forming composition.

The surfactant system can contain an anionic primary surfactant. The article can contain from about 35% to about 100%, alternatively from about 40% to about 90%, alternatively from about 45% to about 85%, alternatively from about 50% to about 80%, alternatively from about 60% to about 75% primary surfactant by weight of the surfactant system on a dry fibrous element basis and/or a dry dissolvable fibrous article basis and/or by weight of the fibrous element-forming composition.

The primary surfactant can be an anionic surfactant with two or more negatively charged hydrophilic groups, particularly, two negatively charged hydrophilic groups where the surfactant is substantially free of sulfate-based

surfactants. The primary surfactant comprises an anionic surfactant comprising a glutamate surfactant selected from the group consisting of sodium cocoyl glutamate, disodium cocoyl glutamate, potassium cocoyl glutamate, dipotassium cocoyl glutamate, ammonium cocoyl glutamate, diammonium cocoyl glutamate, TEA-cocoyl glutamate, or combinations thereof or an alaninate surfactant selected from sodium cocoyl alaninate, sodium lauroyl alaninate, sodium N-dodecanoyl-1-alaninate, and combinations thereof. The primary surfactant can include disodium cocoyl glutamate, disodium laureth sulfosuccinate, disodium cocoamphodiacetate, disodium lauroamphodiacetate, and combinations thereof.

The primary anionic surfactant can comprise at least one glutamate surfactant. Non-limiting examples of glutamate surfactants can include sodium cocoyl glutamate, disodium cocoyl glutamate, potassium cocoyl glutamate, dipotassium cocoyl glutamate, ammonium cocoyl glutamate, diammonium cocoyl glutamate, sodium lauroyl glutamate, disodium lauroyl glutamate, potassium lauroyl glutamate, dipotassium lauroyl glutamate, sodium capryloyl glutamate, disodium capryloyl glutamate, potassium capryloyl glutamate, dipotassium capryloyl glutamate, sodium undecylenoyl glutamate, disodium undecylenoyl glutamate, potassium undecylenoyl glutamate, dipotassium undecylenoyl glutamate, disodium hydrogenated tallowoyl glutamate, sodium stearoyl glutamate, disodium stearoyl glutamate, potassium stearoyl glutamate, dipotassium stearoyl glutamate, sodium myristoyl glutamate, disodium myristoyl glutamate, potassium myristoyl glutamate, dipotassium myristoyl glutamate, sodium cocoyl/hydrogenated tallowoyl glutamate, sodium cocoyl/palmoyl/sunfloweroyl glutamate, sodium hydrogenated tallowoyl glutamate, sodium olivoyl glutamate, disodium olivoyl glutamate, sodium palmoyl glutamate, disodium palmoyl glutamate, TEA-cocoyl glutamate, TEA-hydrogenated tallowoyl glutamate, TEA-lauroyl glutamate, and mixtures thereof.

The at least one glutamate surfactant may be selected from the group consisting of sodium cocoyl glutamate, disodium cocoyl glutamate, potassium cocoyl glutamate, dipotassium cocoyl glutamate, ammonium cocoyl glutamate, diammonium cocoyl glutamate, sodium lauroyl glutamate, disodium lauroyl glutamate, potassium lauroyl glutamate, dipotassium lauroyl glutamate, sodium capryloyl glutamate, disodium capryloyl glutamate, potassium capryloyl glutamate, dipotassium capryloyl glutamate, sodium stearoyl glutamate, disodium stearoyl glutamate, potassium stearoyl glutamate, dipotassium stearoyl glutamate, sodium myristoyl glutamate, disodium myristoyl glutamate, potassium myristoyl glutamate, dipotassium myristoyl glutamate, TEA-cocoyl glutamate, and mixtures thereof

In some examples, the at least one glutamate surfactant may be selected from the group consisting of sodium cocoyl glutamate, disodium cocoyl glutamate, potassium cocoyl glutamate, dipotassium cocoyl glutamate, ammonium cocoyl glutamate, diammonium cocoyl glutamate, TEA-cocoyl glutamate, and mixtures thereof.

The total level of the at least one glutamate surfactant may be from about 8% to about 100%, alternatively from about 8% to about 85%, alternatively from about 12% to about 70%, alternatively from about 17% to about 55%, and alternatively from about 20% to about 45%, by weight of the article. The glutamate level can be by weight on a dry fibrous element basis and/or a dry dissolvable fibrous article basis and/or by weight of the fibrous element-forming composition. The total level of the at least one glutamate surfactant can be from about 40% to about 100%, alternatively from about 40% to about 85%, alternatively from about 45% to about 80%, alternatively from about 50% to about 75%, by weight of the surfactant system on a dry fibrous element basis and/or a dry dissolvable fibrous article basis and/or by weight of the fibrous element-forming composition.

The one or more surfactants of the one or more active agents may also comprise a co-surfactant by weight of the composition, wherein the co-surfactant can be selected from the group consisting of an additional anionic surfactant, a non-ionic surfactant, an amphoteric surfactant, a zwitterionic surfactant, and mixtures thereof.

The article can optionally contain a co-surfactant. The total level of the co-surfactant can be from about 0.5% to about 50%, alternatively from about 2% to about 30%, alternatively from about 5% to about 25%, alternatively from about 7% to about 20%, by weight of the article on a dry fibrous element basis and/or a dry dissolvable fibrous article basis and/or by weight of the fibrous element-forming composition.

The total level of the co-surfactant can be from about 10% to about 65%, alternatively from about 15% to about 55%, alternatively from about 23% to about 50%, by weight of the surfactant system on a dry fibrous element basis and/or a dry dissolvable fibrous article basis and/or by weight of the fibrous element-forming composition.

The additional anionic surfactant may be selected from the group consisting of an isethionate surfactant, a sarcosinate surfactant, a glycinate surfactant, an alaniate surfactant, a sulfosuccinate surfactant, a sulfonate surfactant, a sulfoacetate surfactant, a glucose carboxylate surfactant, an alkyl ether carboxylate surfactant, a taurate surfactant, and mixtures thereof. Each anionic surfactant just listed above will be described in more details below.

The one or more surfactants of the one or more active agents may also comprise at least one isethionate surfactant according to the general formula (II):

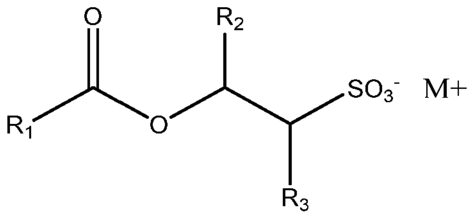

(II)

wherein $R_1$ can be a saturated or unsaturated, straight or branched, alkyl or alkenyl chain with from 6 to 30 carbon atoms, alternatively from 8 to 22 carbon atoms, alternatively from 9 to 18 carbon atoms, $R_2$ and $R_3$ are each independently H or ($C_1$-$C_4$) alkyl, alternatively wherein ($C_1$-$C_4$) alkyl can be methyl, and $M^+$ can be an alkali metal, alternatively lithium, sodium, potassium; or $M^+$ can be an alkali-earth metal, alternatively magnesium; or $M^{+ \text{can be}}$ an ammonium or a substituted ammonium cation.

The isethionate surfactant may be selected from the group consisting of sodium lauroyl isethionate, sodium lauroyl methyl isethionate, sodium oleoyl isethionate, sodium oleoyl methyl isethionate, sodium stearoyl isethionate, sodium stearoyl methyl isethionate, sodium myristoyl isethionate, sodium myristoyl methyl isethionate, sodium palmitoyl isethionate, sodium palmitoyl methyl isethionate, sodium cocoyl isethionate, sodium cocoyl methyl isethionate, a blend of stearic acid and sodium cocoyl isethionate, ammonium cocoyl isethionate, ammonium cocoyl methyl isethionate, and mixtures thereof.

The isethionate surfactant may be selected from the group consisting of sodium lauroyl isethionate, sodium lauroyl methyl isethionate, sodium oleoyl isethionate, sodium stearoyl isethionate, sodium myristoyl isethionate, sodium palmitoyl isethionate, sodium cocoyl isethionate, ammonium cocoyl isethionate, and mixtures thereof.

The isethionate surfactant may be selected from the group consisting of sodium lauroyl isethionate, sodium lauroyl methyl isethionate, sodium stearoyl isethionate, sodium myristoyl isethionate, sodium cocoyl isethionate, ammonium cocoyl isethionate, and mixtures thereof.

The isethionate surfactant may be selected from the group consisting of sodium lauroyl isethionate, sodium cocoyl isethionate, ammonium cocoyl isethionate, and mixtures thereof.

Corresponding commercial products are available, for example, from the company Innospec under the trade name "Iselux®" and from Clariant or Uniquema under the trade names "Hostapon®" or "Arlatone®". Examples of other commercial fatty acyl isethionates that may be used can be Hostapon® surfactants from Clariant such as for sodium cocoyl isethionate: Hostapon® SCI-85C, Hostapon® SCI-78C, or a blend of stearic acid with sodium cocoyl isethionate: Hostapon® SCI-65C. Examples of other commercial fatty acyl isethionates that may be used can be "Jordapon®" surfactants from BASF such as Jordapon® CI prill or Jordapon® CI65; and sodium cocoyl isethionate from Yongan Daily Chemical Co. such as YA-SCI-85® or YA-SCI-65®.

The sarcosinate surfactant may have the general formula (III):

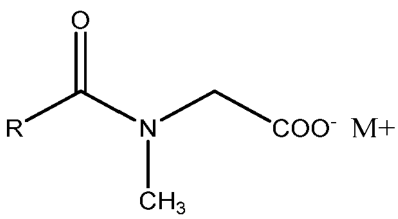

(III)

wherein R can be a saturated or unsaturated, straight or branched alkyl or alkenyl, alternatively alkyl chain with 7 to 17 carbon atoms, alternatively with 9 to 13 carbon atoms and $M^+$ can be H, a sodium, potassium, ammonium or triethanolammonium cation.

The sarcosinate surfactant may be selected from the group consisting of sodium lauroyl sarcosinate, sodium cocoyl sarcosinate, sodium myristoyl sarcosinate, TEA-cocoyl sarcosinate, ammonium cocoyl sarcosinate, ammonium lauroyl sarcosinate, dimer dilinoleyl bis-lauroyl glutamate/lauroyl sarcosinate, disodium lauroamphodiacetate, lauroyl sarcosinate, isopropyl lauroyl sarcosinate, potassium cocoyl sarcosinate, potassium lauroyl sarcosinate, sodium cocoyl sarcosinate, sodium lauroyl sarcosinate, sodium myristoyl sarcosinate, sodium oleoyl sarcosinate, sodium palmitoyl sarcosinate, TEA-cocoyl sarcosinate, TEA-lauroyl sarcosinate, TEA-oleoyl sarcosinate, TEA-palm kernel sarcosinate, and mixtures thereof.

Alternatively, the sarcosinate surfactant may be selected from the group consisting of sodium lauroyl sarcosinate, sodium myristoyl sarcosinate, sodium cocoyl sarcosinate, and mixtures thereof.

The glycinate surfactant may be selected from the group consisting of sodium cocoyl glycinate, sodium lauroyl

glycinate, and mixture thereof.

The alaninate surfactant may be selected from the group consisting of sodium cocoyl alaninate, sodium lauroyl alaninate, sodium N-dodecanoyl-1-alaninate, and mixture thereof.

The sulfosuccinate surfactant may be selected from the group consisting of disodium N-octadecyl sulfosuccinate, disodium lauryl sulfosuccinate, diammonium lauryl sulfosuccinate, sodium lauryl sulfosuccinate, disodium laureth sulfosuccinate, tetrasodium N-(1,2-dicarboxyethyl)-N-octadecyl sulfosuccinnate, diamyl ester of sodium sulfosuccinic acid, dihexyl ester of sodium sulfosuccinic acid, dioctyl esters of sodium sulfosuccinic acid, and mixtures thereof.

The sulfonate surfactant may be selected from the group consisting of alpha olefin sulfonates, linear alkylbenzene sulfonates, sodium laurylglucosides hydroxypropylsulfonate, and mixtures thereof.

The sulfoacetate surfactant may be selected from the group consisting of sodium lauryl sulfoacetate, ammonium lauryl sulfoacetate, and mixture thereof.

The glucose carboxylate surfactant may be selected from the group consisting of sodium lauryl glucoside carboxylate, sodium cocoyl glucoside carboxylate, and mixtures thereof.

The alkyl ether carboxylate surfactant may be selected from the group consisting of sodium laureth-4 carboxylate, laureth-5 carboxylate, laureth-13 carboxylate, sodium C12-13 pareth-8 carboxylate, sodium C12-15 pareth-8 carboxylate and mixtures thereof.

The taurate surfactant may be selected from the group consisting of sodium methyl cocoyl taurate, sodium methyl lauroyl taurate, sodium methyl oleoyl taurate, and mixtures thereof.

The anionic surfactant being not a glutamate surfactant may comprise a lactate or lactylate. Non-limiting example of lactates can include sodium lactate. Non-limiting examples of lactylates can include sodium lauroyl lactylate, sodium cocoyl lactylate, and mixture thereof.

The total level of additional anionic surfactant may be from about 0% to about 20% by weight of the fibrous element-forming composition or on a dry fibrous element basis and/or a dry dissolvable fibrous article basis. Alternatively, the total level of the anionic surfactant being not a glutamate surfactant may be from about 0.5% to about 15% by weight of the fibrous element-forming composition or on a dry fibrous element basis and/or a dry dissolvable fibrous article basis. The one or more surfactants of the one or more active agents may comprise a non-ionic surfactant. The non-ionic surfactant may be selected from the group consisting alkyl polyglucoside, alkyl glycoside, acyl glucamide and mixtures thereof.

In that case, alkyl can be defined as a saturated or unsaturated, straight or branched alkyl chain with 6 to 30 carbon atoms, alternatively with 8 to 22 carbon atoms, alternatively with 9 to 18 carbon atoms. In that case, acyl can be defined as of formula R-C(O)-, wherein R can be a saturated or unsaturated, straight or branched alkyl or alkenyl, alternatively alkyl chain with 6 to 30 carbon atoms, alternatively with 8 to 22 carbon atoms, alternatively with 9 to 18 carbon atoms.

The alkyl glucoside may be selected from the group consisting of decyl glucoside, cocoyl glucoside, lauroyl glucoside, and mixtures thereof.

The acyl glucamide may be selected from the group consisting of lauroyl/myristoyl methyl glucamide, capryloyl/capryloyl methyl glucamide, cocoyl methyl glucamide and mixtures thereof. Alternatively, the non-ionic surfactant may be selected from the group consisting of cocoamide monoethanolamine, lauramide monoethanolamine, cocoyl glucoside, lauroyl glucoside, decyl glucoside, and mixtures thereof.

The total level of the non-ionic surfactant may be from about 0% to about 25% by weight of the fibrous element-forming composition or on a dry fibrous element basis and/or a dry dissolvable fibrous article basis. Alternatively, the total level of the non-ionic surfactant may be from about 0.1% to about 15% by weight of the fibrous element-forming composition or on a dry fibrous element basis and/or a dry dissolvable fibrous article basis. Alternatively, the total level of the non-ionic surfactant may be from about 0.5% to about 10% by weight of the fibrous element-forming composition or on a dry fibrous element basis and/or a dry dissolvable fibrous article basis. Suitable amphoteric or zwitterionic surfactants can include those described in U.S. Patent No. 5,104,646 and U.S. Patent No. 5,106,609.

Amphoteric surfactants can include those that can be broadly described as derivatives of aliphatic secondary and tertiary amines in which an aliphatic radical can be straight or branched chain and wherein an aliphatic substituent can contain from 8 to 18 carbon atoms such that one carbon atom can contain an anionic water solubilizing group, e.g., carboxy, sulfonate, phosphate, or phosphonate. Examples of compounds falling within this definition can be sodium 3-dodecyl-aminopropionate, sodium 3-dodecylaminopropane sulfonate, N-alkyltaurines such as the one prepared by reacting dodecylamine with sodium isethionate according to the teaching of U.S. Patent No. 2,658,072, N-higher alkyl aspartic acids such as those produced according to the teaching of U.S. Patent No. 2,438,091, and products described in U.S. Patent No. 2,528,378.

The amphoteric surfactant described herein may be selected from the group consisting of sodium lauroamphoacetate, sodium cocoamphoacetate, disodium lauroamphodiacetate, disodium cocodiamphoacetate, and mixtures thereof.

Zwitterionic surfactants suitable for use in the co-surfactants of the one or more active agents described herein

may include those that are broadly described as derivatives of aliphatic quaternary ammonium, phosphonium, and sulfonium compounds, in which the aliphatic radicals can be straight or branched chains, and wherein one of the aliphatic substituents can contain from 8 to 18 carbon atoms and one can contain an anionic group, e.g., carboxy, sulfonate, phosphate, or phosphonate.

Hence, the one or more surfactants of the one or more active agents may comprise at least an amphoteric or zwitterionic surfactant selected from the group consisting of cocamidopropyl betaine, lauramidopropyl betaine, coco-betaine, lauryl betaine, lauryl hydroxysultaine, cocamidopropyl hydroxysultaine, coco-hydroxysultaine, coco-sultaine, lauryl sultaine, sodium cocoamphoacetate, disodium cocoamphodiacetate, sodium lauroamphoacetate, disodium lauroamphodiacetate, lauramine oxide, lauryl hydroxysultaine, and mixtures thereof.

Examples of betaine zwitterionic surfactants may include coco dimethyl carboxymethyl betaine, cocoamidopropyl betaine (CAPB), coco-betaine, lauryl amidopropyl betaine (LAPB), oleyl betaine, lauryl dimethyl carboxymethyl betaine, lauryl dimethyl alpha-carboxyethyl betaine, cetyl dimethyl carboxymethyl betaine, lauryl bis-(2-hydroxyethyl) carboxymethyl betaine, stearyl bis-(2-hydroxypropyl) carboxymethyl betaine, oleyl dimethyl gamma-carboxypropyl betaine, lauryl bis-(2-hydroxypropyl)alpha-carboxyethyl betaine, and mixtures thereof. Examples of sulfobetaines may include coco dimethyl sulfopropyl betaine, stearyl dimethyl sulfopropyl betaine, lauryl dimethyl sulfoethyl betaine, lauryl bis-(2-hydroxyethyl) sulfopropyl betaine, and mixtures thereof.

The total level of the zwitterionic surfactant may be from about 0.5% to about 20% by weight of the fibrous element-forming composition or on a dry fibrous element basis and/or a dry dissolvable fibrous article basis. Alternatively, the total level of the non-ionic surfactant may be from about 2% to about 15% by weight of the fibrous element-forming composition or on a dry fibrous element basis and/or a dry dissolvable fibrous article basis. Alternatively, the total level of the non-ionic surfactant may be from about 4% to about 13% by weight of the fibrous element-forming composition or on a dry fibrous element basis and/or a dry dissolvable fibrous article basis.

Cationic Polymers

The fibrous article can contain from about 0.05% to about 5% cationic polymer, from about 0.1% to about 2% cationic polymer, from about 0.2% to about 1.5% cationic polymer, from about 0.3% to about 1.0% cationic polymer, from about 0.4% to about 0.75% cationic polymer, by weight of the fibrous element-forming composition or on a dry fibrous element basis and/or a dry dissolvable fibrous article basis.

The cationic polymers can have a weight average molecular weight from about 500,000 g/mol to about 2.5 million g/mol, alternatively from about 500,000 g/mol to about 2 million g/mol, alternatively from about 500,000 g/mol to about 1.5 million, alternatively about 500,000 g/mol to about 1 million as measured by gel permeation chromatography. The cationic polymers can have a weight average molecular weight greater than 500,000 g/mol, alternatively greater than 1 million g/mol as measured by gel permeation chromatography.

The cationic polymers can have a weight average charge density greater than 0.2 meq/g, alternatively greater than 0.4 meq/g, alternatively 0.6 meg/g, alternatively 0.8 meg/g, alternatively 1 meq/g, alternatively 1.2 meq/g, alternatively 1.5 meg/g, alternatively 2 meg/g, alternatively greater than 3 meg/g, alternatively greater than 5 meg/g as measured according to the Charge Density Test Method. The cationic polymers can have a weight average charge density from about 0.4 meg/g to about 5 meg/g, alternatively from about 1 meg/g to about 3 meg/g, alternatively from about 1 meg/g to about 2.5 meg/g as measured according to the Charge Density Test Method.

Cationic Guar Polymer

The hair care composition can comprise (a) a cationic guar polymer. Cationic guar polymers are cationically substituted galactomannan (guar) gum derivatives. Guar gum for use in preparing these guar gum derivatives is typically obtained as a naturally occurring material from the seeds of the guar plant. The guar molecule itself is a straight chain mannan, which is branched at regular intervals with single membered galactose units on alternative mannose units. The mannose units are linked to each other by means of $\beta$(1-4) glycosidic linkages. The galactose branching arises by way of an $\alpha$(1-6) linkage. Cationic derivatives of the guar gums are obtained by reaction between the hydroxyl groups of the polygalactomannan and reactive quaternary ammonium compounds. The degree of substitution of the cationic groups onto the guar structure should be sufficient to provide the requisite cationic charge density described above.

The cationic guar polymer can have a weight average M.Wt. of less than 2.2 million g/mol, or from about 150 thousand g/mol to about 2 million g/mol, or from about 200 thousand to about 1.9 million g/mol, or from about 300 thousand to about 1.8 million g/mol, or from about 400 thousand to about 1.7 million g/mol, or from about 500,000 g/mol to about 1.6 million g/mol. The cationic guar polymer can have a weight average M.Wt. of greater than about 150,000 g/mol, alternatively greater than about 1 million g/mol, alternatively greater than about 1.5 million g/mol, alternatively greater than about 2 million g/mol, and alternatively greater than about 2.5 million g/mol.

The cationic guar polymer can have a weight average charge density of from about 0.2 meq/g to about 2.2 meg/g,

or from about 0.3 meq/g to about 2.0 meg/g, or from about 0.4 meq/g to about 1.9 meg/g, or from about 0.5 meq/g to about 1.8 meg/g, or from about 0.6 meq/g to about 1.7 meg/g, or from about 0.6 meq/g to about 1.5 meq/g, or from about 0.6 meq/g to about 1.3 meg/g, and/or from about 0.7 meq/g to about 1.0 meg/g.

The cationic guar polymer may be formed from quaternary ammonium compounds. The quaternary ammonium compounds for forming the cationic guar polymer can conform to the general formula 1:

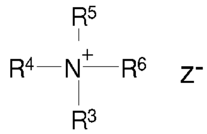

wherein where $R^3$, $R^4$ and $R^5$ are methyl or ethyl groups; $R^6$ is either an epoxyalkyl group of the general formula 2:

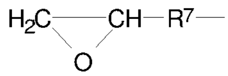

or $R^6$ is a halohydrin group of the general formula 3:

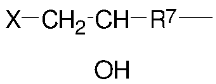

wherein $R^7$ is a $C_1$ to $C_3$ alkylene; X is chlorine or bromine, and Z is an anion such as Cl-, Br-, I- or $HSO_4$-.
The cationic guar polymer can conform to the general formula 4:

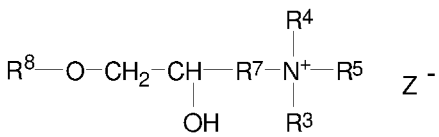

wherein $R^8$ is guar gum; and wherein $R^4$, $R^5$, $R^6$ and $R^7$ are as defined above; and wherein Z is a halogen. The cationic guar polymer can conform to Formula 5:

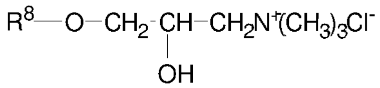

Suitable cationic guar polymers can include cationic guar gum derivatives, such as guar hydroxypropyltrimonium chloride. The cationic guar polymer is a guar hydroxypropyltrimonium chloride. Specific examples of guar hydroxypropyltrimonium chlorides include the Jaguar® series commercially available from Rhone-Poulenc Incorporated, for example Jaguar® C-500, commercially available from Rhodia. Jaguar® C-500 has a charge density of 0.8 meq/g and a weight average molecular weight of 500,000 g/mol. Another guar hydroxypropyltrimonium chloride with a charge density of about 1.1 meq/g and a weight average molecular weight of about 500,000 g/mol is available from Ashland. A further guar hydroxypropyltrimonium chloride with a charge density of about 1.5 meq/g and a weight average molecular weight of about 500,000 g/mole is available from Ashland.

Other suitable guar hydroxypropyltrimonium chloride are: Hi-Care 1000, which has a charge density of about 0.7 meq/g and a weight average molecular weight of about 600,000 g/mole is available from Rhodia; N-Hance 3269 and N-Hance 3270, which have a charge density of about 0.7 meq/g and a weight average molecular weight of about 425,000 g/mol are available from Ashland; N-Hance 3271 which has a charge density of about 0.7 meq/g and a weight average molecular weight of about 500,000 g/mol and is available from Ashland; BF-13, which is a borate (boron) free guar of charge density of about 1.1 meq/g and weight average molecular weight of about 800,000 and BF-17, which is a borate (boron) free guar of charge density of about 1.7 meq/g and M. W.t of about 800,000 both available from Ashland; N-Hance CG17 has a charge density of about 1.0meq/g and a weight average molecular weight of about 1,600,000 g/mol and is available from Ashland; and N-Hance 3196 has a charge density of about 0.7 meq/g and a weight average molecular weight of 1,700,000 g/mol and is available from Ashland.

Cationic Synthetic Polymer

The hair care composition can include (b) a cationic synthetic polymer, wherein the cationic synthetic polymer can have a weight average M.Wt. of from about 1,000 g/mol to about 2.0 million g/mol, and wherein the cationic guar polymer can have a charge density of from about 2 meq/g to about 10 meq/g. The hair care composition can comprise a cationic synthetic polymer from about 0.01% to about 2.5% by total weight of the composition.

The cationic synthetic polymers may be formed from

i) one or more cationic monomer units, and optionally
ii) one or more monomer units bearing a negative charge, and/or
iii) a nonionic monomer,

wherein the subsequent charge of the copolymer is positive. The ratio of the three types of monomers is given by "m", "p" and "q" where "m" is the number of cationic monomers, "p" is the number of monomers bearing a negative charge and "q" is the number of nonionic monomers

The cationic polymers can be water soluble or dispersible, non-crosslinked, and cationic synthetic polymers having the following structure:

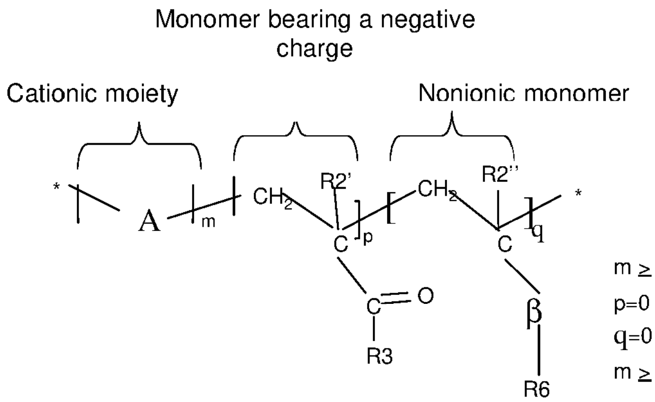


$m \geq 1$
p=0 or $\geq 1$
q=0 or $\geq 1$
$m \geq p$

where A, may be one or more of the following cationic moieties:

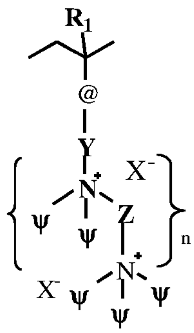
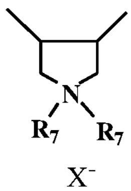
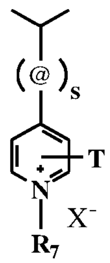

where @ = amido, alkylamido, ester, ether, alkyl or alkylaryl;
where Y = C1-C22 alkyl, alkoxy, alkylidene, alkyl or aryloxy;
where $\psi$ = C1-C22 alkyl, alkyloxy, alkyl aryl or alkyl arylox;.
where Z = C1-C22 alkyl, alkyloxy, aryl or aryloxy;
where R1 = H, C1-C4 linear or branched alkyl;
where s = 0 or 1, n = 0 or $\geq$ 1;
where T and R7 = C1-C22 alkyl; and
where X- = halogen, hydroxide, alkoxide, sulfate or alkylsulfate.

Where the monomer bearing a negative charge is defined by R2' = H, C1-C4 linear or branched alkyl and R3 as:

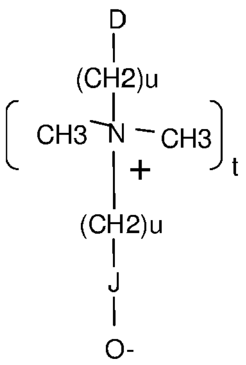 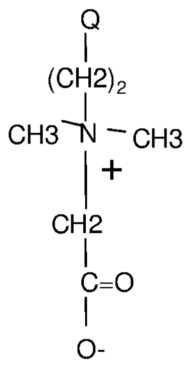 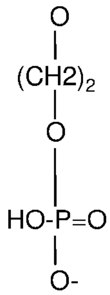 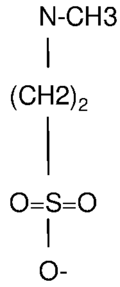

where D = O, N, or S;
where Q = $NH_2$ or O;
where u = 1-6;
where t = 0-1; and
where J = oxygenated functional group containing the following elements P, S, C.
Where the nonionic monomer is defined by R2" = H, C1-C4 linear or branched alkyl, R6 = linear or branched alkyl, alkyl aryl, aryl oxy, alkyloxy, alkylaryl oxy and β is defined as

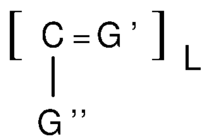

;

and
where G' and G" are, independently of one another, O, S or N-H and L =0 or 1.

Examples of cationic monomers include aminoalkyl (meth)acrylates, (meth)aminoalkyl (meth)acrylamides; monomers comprising at least one secondary, tertiary or quaternary amine function, or a heterocyclic group containing a nitrogen atom, vinylamine or ethylenimine; diallyldialkyl ammonium salts; their mixtures, their salts, and macromonomers deriving from therefrom.
Further examples of cationic monomers include dimethylaminoethyl (meth)acrylate, dimethylaminopropyl (meth)acrylate, ditertiobutylaminoethyl (meth)acrylate, dimethylaminomethyl (meth)acrylamide, dimethylaminopropyl (meth)acrylamide, ethylenimine, vinylamine, 2-vinylpyridine, 4- vinylpyridine, trimethylammonium ethyl (meth)acrylate chloride, trimethylammonium ethyl (meth)acrylate methyl sulphate, dimethylammonium ethyl (meth)acrylate benzyl chloride, 4-benzoylbenzyl dimethylammonium ethyl acrylate chloride, trimethyl ammonium ethyl (meth)acrylamido chloride, trimethyl ammonium propyl (meth)acrylamido chloride, vinylbenzyl trimethyl ammonium chloride, diallyldimethyl ammonium chloride.
Suitable cationic monomers include those which comprise a quaternary ammonium group of formula $-NR_3^+$, wherein R, which is identical or different, represents a hydrogen atom, an alkyl group comprising 1 to 10 carbon atoms, or a benzyl group, optionally carrying a hydroxyl group, and comprise an anion (counter-ion). Examples of anions are halides such as chlorides, bromides, sulphates, hydrosulphates, alkylsulphates (for example comprising 1 to 6 carbon atoms), phosphates, citrates, formates, and acetates.
Suitable cationic monomers include trimethylammonium ethyl (meth)acrylate chloride, trimethylammonium ethyl (meth)acrylate methyl sulphate, dimethylammonium ethyl (meth)acrylate benzyl chloride, 4-benzoylbenzyl dimethylammonium ethyl acrylate chloride, trimethyl ammonium ethyl (meth)acrylamido chloride, trimethyl ammonium propyl (meth)acrylamido chloride, vinylbenzyl trimethyl ammonium chloride.
Additional suitable cationic monomers include trimethyl ammonium propyl (meth)acrylamido chloride.
Examples of monomers bearing a negative charge include alpha ethylenically unsaturated monomers comprising a phosphate or phosphonate group, alpha ethylenically unsaturated monocarboxylic acids, monoalkylesters of alpha ethylenically unsaturated dicarboxylic acids, monoalkylamides of alpha ethylenically unsaturated dicarboxylic acids, alpha ethylenically unsaturated compounds comprising a sulphonic acid group, and salts of alpha ethylenically unsaturated compounds comprising a sulphonic acid group.
Suitable monomers with a negative charge include acrylic acid, methacrylic acid, vinyl sulphonic acid, salts of vinyl sulfonic acid, vinylbenzene sulphonic acid, salts of vinylbenzene sulphonic acid, alpha-acrylamidomethylpropanesulphonic acid, salts of alpha-acrylamidomethylpropanesulphonic acid, 2-sulphoethyl methacrylate, salts of 2-sulphoethyl methacrylate, acrylamido-2-methylpropanesulphonic acid (AMPS), salts of acrylamido-2-methylpropanesulphonic acid, and styrenesulphonate (SS).

Examples of nonionic monomers include vinyl acetate, amides of alpha ethylenically unsaturated carboxylic acids, esters of an alpha ethylenically unsaturated monocarboxylic acids with an hydrogenated or fluorinated alcohol, polyethylene oxide (meth)acrylate (i.e. polyethoxylated (meth)acrylic acid), monoalkylesters of alpha ethylenically unsaturated dicarboxylic acids, monoalkylamides of alpha ethylenically unsaturated dicarboxylic acids, vinyl nitriles, vinylamine amides, vinyl alcohol, vinyl pyrolidone, and vinyl aromatic compounds.

Suitable nonionic monomers include styrene, acrylamide, methacrylamide, acrylonitrile, methylacrylate, ethylacrylate, n-propylacrylate, n-butylacrylate, methylmethacrylate, ethylmethacrylate, n-propylmethacrylate, n-butylmethacrylate, 2-ethyl-hexyl acrylate, 2-ethyl-hexyl methacrylate, 2-hydroxyethylacrylate and 2-hydroxyethylmethacrylate.

The anionic counterion ( X- ) in association with the cationic synthetic polymers may be any known counterion so long as the polymers remain soluble or dispersible in water, in the hair care composition, or in a coacervate phase of the hair care composition, and so long as the counterions are physically and chemically compatible with the essential components of the hair care composition or do not otherwise unduly impair product performance, stability or aesthetics. Non limiting examples of such counterions include halides (e.g., chlorine, fluorine, bromine, iodine), sulfate and methylsulfate.

The cationic synthetic polymer can have a weight average M.Wt. of from about 1,500 g/mol to about 1.8 million g/mol, or from about 2,000 g/mol to about 1.7 million g/mol, or from about 3,000 g/mol to about 1.6 million g/mol, or from about 4,000 g/mol to about 1.5 million g/mol, or from about 5,000 g/mol to about 1.6 million g/mol, or from about 6,000 g/mol to about 1.5 million g/mol, or from about 7,000 g/mol to about 1.4 million g/mol, or from about 8,000 g/mol to about 1.4 million g/mol, or from about 9,000 g/mol to about 1.3 million g/mol, or from about 10,000 g/mol to about 1.2 million g/mol or from about 11,000 g/mol to about 1.1 million g/mol, or from about 25,000 g/mol to about 750,000 g/mol, or from about 50,000 g/mol to about 500,000 g/mol, or from about 75,000 g/mol to about 300,000 g/mol, and/or from about 100,000 g/mol to about 200,000 g/mol.

The cationic synthetic polymer can have a weight average charge density of from about 2.2 meq/g to about 9.5 meg/g, or from about 2.5 meq/g to about 8 meg/g, or from about 3 meq/g to about 8 meg/g, or from about 3.5 meq/g to about 7.5 meg/g, and/or from about 4 meq/g to about 7 meg/g.

The cationic synthetic polymer can comprise polydiallyldimethylammonium chloride (polyDADMAC). PolyDADMAC is also known as polyquaternium-6. Specific examples of polyDADMAC are Mirapol® 100 series from Solvay, Merquat™ 100 series from Lubrizol and Salcare® SC 30 from BASF. For example, Mirapol® 100s has a charge density of 6.2 meq/g and a weight average molecular weight of 150,000 g/mol, is available from Solvay.

The hair care composition may further comprise (c) a cationic non-guar galactomannan polymer, (d) a cationic starch polymer, (e) a cationic copolymer of acrylamide monomers and cationic monomers, (f) a cationic cellulose polymer or (g) a mixture of such polymers.

Cationic Non-Guar Galactomannan Polymers

The dispersion compositions can comprise a galactomannan polymer derivative having a mannose to galactose ratio of between 5:1 and 1:1 on a monomer to monomer basis, the galactomannan polymer derivative selected from the group consisting of a cationic galactomannan polymer derivative and an amphoteric galactomannan polymer derivative having a net positive charge. As used herein, the term "cationic galactomannan" refers to a galactomannan polymer to which a cationic group is added. The term "amphoteric galactomannan" refers to a galactomannan polymer to which a cationic group and an anionic group are added such that the polymer has a net positive charge.

Galactomannan polymers are present in the endosperm of seeds of the Leguminosae family. Galactomannan polymers are made up of a combination of mannose monomers and galactose monomers. The galactomannan molecule is a straight chain mannan branched at regular intervals with single membered galactose units on specific mannose units. The mannose units are linked to each other by means of $\beta$ (1-4) glycosidic linkages. The galactose branching arises by way of an $\alpha$ (1-6) linkage. The ratio of mannose monomers to galactose monomers varies according to the species of the plant and also is affected by climate. Non Guar Galactomannan polymer derivatives can have a ratio of mannose to galactose of greater than 2:1 on a monomer to monomer basis. Suitable ratios of mannose to galactose can be greater than about 3:1, and the ratio of mannose to galactose can be greater than about 4:1. Analysis of mannose to galactose ratios is well known in the art and is typically based on the measurement of the galactose content.

The gum for use in preparing the non-guar galactomannan polymer derivatives is typically obtained as naturally occurring material such as seeds or beans from plants. Examples of various non-guar galactomannan polymers include but are not limited to Tara gum (3 parts mannose/1 part galactose), Locust bean or Carob (4 parts mannose/1 part galactose), and Cassia gum (5 parts mannose/1 part galactose).

The galactomannan polymer derivative can be a cationic derivative of the non-guar galactomannan polymer, which is obtained by reaction between the hydroxyl groups of the polygalactomannan polymer and reactive quaternary

ammonium compounds. Suitable quaternary ammonium compounds for use in forming the cationic galactomannan polymer derivatives include those conforming to the general formulas 1-5, as defined above.

Cationic non-guar galactomannan polymer derivatives formed from the reagents described above are represented by the general formula 6:

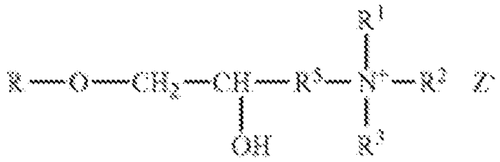

wherein R is the gum. The cationic galactomannan derivative can be a gum hydroxypropyltrimethylammonium chloride, which can be more specifically represented by the general formula 7:

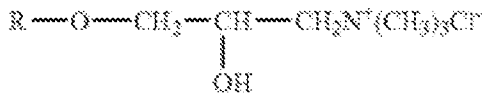

The galactomannan polymer derivative can be an amphoteric galactomannan polymer derivative having a net positive charge, obtained when the cationic galactomannan polymer derivative further comprises an anionic group.

The cationic non-guar galactomannan can have a ratio of mannose to galactose that is greater than about 4:1. The dispersion compositions may comprise a galactomannan polymer derivative, by weight, of the composition. The hair care compositions can comprise from about 0.05% to about 2%, by weight, of the composition, of a galactomannan polymer derivative.

(d) Cationically Modified Starch Polymer

The dispersion compositions can comprise water-soluble cationically modified starch polymers. As used herein, the term "cationically modified starch" refers to a starch to which a cationic group is added prior to degradation of the starch to achieve a relatively small weight average molecular weight, or wherein a cationic group is added after modification of the starch to achieve a desired weight average molecular weight. The definition of the term "cationically modified starch" also includes amphoterically modified starch. The term "amphoterically modified starch" refers to a starch hydrolysate to which a cationic group and an anionic group are added.

The dispersion compositions can comprise cationically modified starch polymers at a range of about 0.01% to about 10%, and/or from about 0.05% to about 5%, by weight, of the composition. The cationically modified starch polymers disclosed herein can have a percent of bound nitrogen of from about 0.5% to about 4%.

The dispersion compositions can include starch polymers that is chemically modified by the addition of amino and/or ammonium groups into the starch molecules. Non-limiting examples of these ammonium groups may include substituents such as hydroxypropyl trimmonium chloride, trimethylhydroxypropyl ammonium chloride, dimethylstearylhydroxypropyl ammonium chloride, and dimethyldodecylhydroxypropyl ammonium chloride. See Solarek, D. B., Cationic Starches in Modified Starches: Properties and Uses, Wurzburg, O. B., Ed., CRC Press, Inc., Boca Raton, Fla. 1986, pp 113-125. The cationic groups may be added to the starch prior to degradation to a relatively small weight average molecular weight or the cationic groups may be added after such modification.

The cationically modified starch polymers can generally have a degree of substitution of a cationic group from about 0.1 to about 7. As used herein, the "degree of substitution" of the cationically modified starch polymers is an average measure of the number of hydroxyl groups on each anhydroglucose unit which is derivatized by substituent groups. Since each anhydroglucose unit has three potential hydroxyl groups available for substitution, the maximum possible degree of substitution is 3. The degree of substitution is expressed as the number of moles of substituent groups per mole of anhydroglucose unit, on a molar average basis. The degree of substitution may be determined using proton nuclear magnetic resonance spectroscopy (".sup.1H NMR") methods well known in the art. Suitable .sup.1H NMR techniques include those described in "Observation on NMR Spectra of Starches in Dimethyl Sulfoxide, Iodine-Complexing, and Solvating in Water-Dimethyl Sulfoxide", Qin-Ji Peng and Arthur S. Perlin, Carbohydrate Research, 160 (1987), 57-72; and "An Approach to the Structural Analysis of Oligosaccharides by NMR Spectroscopy", J. Howard Bradbury and J. Grant Collins, Carbohydrate Research, 71, (1979), 15-25.

The source of starch before chemical modification can be chosen from a variety of sources such as tubers, legumes, cereal, and grains. Non-limiting examples of this source starch may include corn starch, wheat starch, rice starch, waxy corn starch, oat starch, cassaya starch, waxy barley, waxy rice starch, glutenous rice starch, sweet rice

starch, amioca, potato starch, tapioca starch, oat starch, sago starch, sweet rice, or mixtures thereof.

Cationically modified starch polymers can be selected from degraded cationic maize starch, cationic tapioca, cationic potato starch, and mixtures thereof.

The starch, prior to degradation or after modification to achieve a relatively small weight average molecular weight, may comprise one or more additional modifications. For example, these modifications may include cross-linking, stabilization reactions, phosphorylations, and hydrolyzations. Stabilization reactions may include alkylation and esterification.

The cationically modified starch polymers may be incorporated into the composition in the form of hydrolyzed starch (e.g., acid, enzyme, or alkaline degradation), oxidized starch (e.g., peroxide, peracid, hypochlorite, alkaline, or any other oxidizing agent), physically/mechanically degraded starch (e.g., via the thermo-mechanical energy input of the processing equipment), or combinations thereof.

An optimal form of the starch is one which is readily soluble in water and forms a substantially clear (% Transmittance.gtoreq.80 at 600 nm) solution in water. The transparency of the composition is measured by Ultra-Violet/Visible (UV/VIS) spectrophotometry, which determines the absorption or transmission of UV/VIS light by a sample, using a Gretag Macbeth Colorimeter Color i 5 according to the related instructions. A light wavelength of 600 nm has been shown to be adequate for characterizing the degree of clarity of cosmetic compositions.

Suitable cationically modified starch for use in compositions is available from known starch suppliers. Nonionic modified starch that could be further derivatized to a cationically modified starch as is known in the art can be suitable. Other suitable modified starch starting materials may be quaternized, as is known in the art, to produce the cationically modified starch polymer suitable for use in the invention.

Starch Degradation Procedure: A starch slurry is prepared by mixing granular starch in water. The temperature is raised to about 35°C. An aqueous solution of potassium permanganate is then added at a concentration of about 50 ppm based on starch. The pH is raised to about 11.5 with sodium hydroxide and the slurry is stirred sufficiently to prevent settling of the starch. Then, about a 30% solution of hydrogen peroxide diluted in water is added to a level of about 1% of peroxide based on starch. The pH of about 11.5 is then restored by adding additional sodium hydroxide. The reaction is completed over about a 1 to about 20 hour period. The mixture is then neutralized with dilute hydrochloric acid. The degraded starch is recovered by filtration followed by washing and drying.

Cationic copolymer of an Acrylamide Monomer and a Cationic Monomer

The dispersion composition can comprise a cationic copolymer of an acrylamide monomer and a cationic monomer. The cationic copolymer can be a synthetic cationic copolymer of acrylamide monomers and cationic monomers.

The cationic copolymer can comprise:

(i) an acrylamide monomer of the following Formula AM:

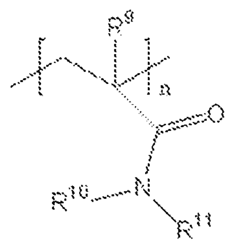

Formula AM

where $R^9$ is H or $C_{1-4}$ alkyl; and $R^{10}$ and $R^{11}$ are independently selected from the group consisting of H, $C_{1-4}$ alkyl, $CH_2OCH_3$, $CH_2OCH_2CH(CH_3)_2$, and phenyl, or together are $C_{3-6}$cycloalkyl; and

(ii) a cationic monomer conforming to Formula CM:

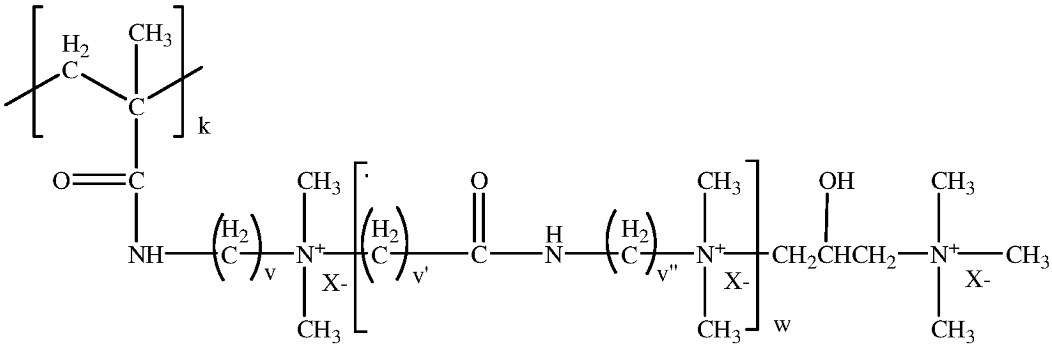

Formula CM

where k = 1, each of v, v', and v" is independently an integer of from 1 to 6, w is zero or an integer of from 1 to 10, and $X^-$ is an anion.

The cationic monomer can conform to Formula CM and where k = 1, v = 3 and w = 0, z = 1 and $X^-$ is $Cl^-$ to form the following structure:

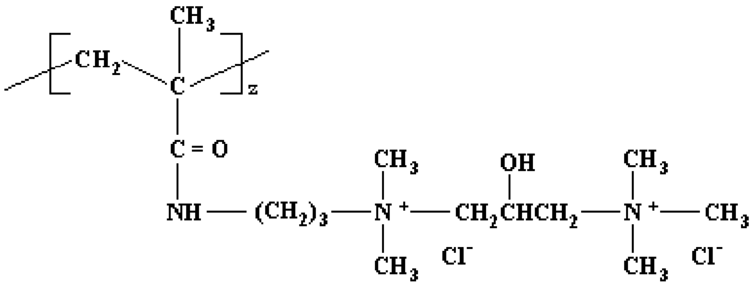

The above structure may be referred to as diquat. The cationic monomer can conform to Formula CM and wherein v and v" are each 3, v' = 1, w =1, y = 1 and $X^-$ is $Cl^-$, such as:

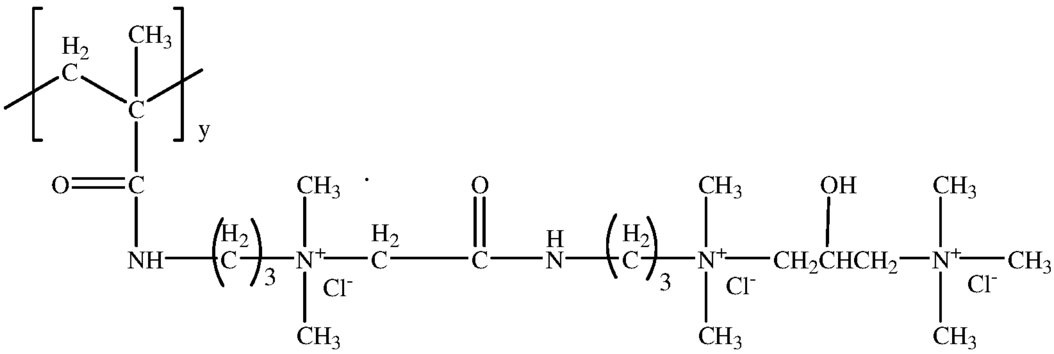

The above structure may be referred to as triquat.

The acrylamide monomer can be either acrylamide or methacrylamide.

The cationic copolymer (b) can be AM:TRIQUAT which is a copolymer of acrylamide and 1,3-Propanediaminium,N-[2-[[[dimethyl[3-[(2-methyl-1-oxo-2-propenyl)amino]propyl]ammonio]acetyl]amino]ethyl]2-hydroxy-N,N,N',N',N'-pentamethyl-, trichloride. AM:TRIQUAT is also known as polyquaternium 76 (PQ76). AM:TRIQUAT may have a charge density of 1.6 meq/g and a M.Wt. of 1.1 million g/mol.

The cationic copolymer can be an acrylamide monomer and a cationic monomer, wherein the cationic monomer is selected from the group consisting of: dimethylaminoethyl (meth)acrylate, dimethylaminopropyl (meth)acrylate, ditertiobutylaminoethyl (meth)acrylate, dimethylaminomethyl (meth)acrylamide, dimethylaminopropyl (meth)acrylamide; ethylenimine, vinylamine, 2-vinylpyridine, 4- vinylpyridine; trimethylammonium ethyl (meth)acrylate chloride, trimethylammonium ethyl (meth)acrylate methyl sulphate, dimethylammonium ethyl (meth)acrylate benzyl chloride, 4-benzoylbenzyl dimethylammonium ethyl acrylate chloride, trimethyl ammonium ethyl (meth)acrylamido chloride, trimethyl ammonium propyl (meth)acrylamido chloride, vinylbenzyl trimethyl ammonium chloride, diallyldimethyl ammonium

chloride, and mixtures thereof.

The cationic copolymer comprises a cationic monomer selected from the group consisting of: cationic monomers include trimethylammonium ethyl (meth)acrylate chloride, trimethylammonium ethyl (meth)acrylate methyl sulphate, dimethylammonium ethyl (meth)acrylate benzyl chloride, 4-benzoylbenzyl dimethylammonium ethyl acrylate chloride, trimethyl ammonium ethyl (meth)acrylamido chloride, trimethyl ammonium propyl (meth)acrylamido chloride, vinylbenzyl trimethyl ammonium chloride, and mixtures thereof.

The cationic copolymer can be water-soluble. The cationic copolymer can be formed from (1) copolymers of (meth)acrylamide and cationic monomers based on (meth)acrylamide, and/or hydrolysis-stable cationic monomers, (2) terpolymers of (meth)acrylamide, monomers based on cationic (meth)acrylic acid esters, and monomers based on (meth) acrylamide, and/or hydrolysis-stable cationic monomers. Monomers based on cationic (meth)acrylic acid esters may be cationized esters of the (meth)acrylic acid containing a quaternized N atom. Cationized esters of the (meth)acrylic acid containing a quaternized N atom can be quaternized dialkylaminoalkyl (meth)acrylates with C1 to C3 in the alkyl and alkylene groups. The cationized esters of the (meth)acrylic acid containing a quaternized N atom are selected from the group consisting of: ammonium salts of dimethylaminomethyl (meth)acrylate, dimethylaminoethyl (meth)acrylate, dimethylaminopropyl (meth)acrylate, diethylaminomethyl (meth)acrylate, diethylaminoethyl (meth)acrylate; and diethylaminopropyl (meth)acrylate quaternized with methyl chloride. The cationized esters of the (meth)acrylic acid containing a quaternized N atom can be dimethylaminoethyl acrylate, which may be quaternized with an alkyl halide, or with methyl chloride or benzyl chloride or dimethyl sulfate (ADAME-Quat). The cationic monomer when based on (meth)acrylamides can be quaternized dialkylaminoalkyl(meth)acrylamides with C1 to C3 in the alkyl and alkylene groups, or dimethylaminopropylacrylamide, which is quaternized with an alkyl halide, or methyl chloride or benzyl chloride or dimethyl sulfate.

The cationic monomer based on a (meth)acrylamide is a quaternized dialkylaminoalkyl(meth)acrylamide with C1 to C3 in the alkyl and alkylene groups. The cationic monomer based on a (meth)acrylamide is dimethylaminopropylacrylamide, which is quaternized with an alkyl halide, especially methyl chloride or benzyl chloride or dimethyl sulfate.

The cationic monomer is a hydrolysis-stable cationic monomer. Hydrolysis-stable cationic monomers can be, in addition to a dialkylaminoalkyl(meth)acrylamide, all monomers that can be regarded as stable to the OECD hydrolysis test. The cationic monomer is hydrolysis-stable and the hydrolysis-stable cationic monomer is selected from the group consisting of: diallyldimethylammonium chloride and water-soluble, cationic styrene derivatives.

The cationic copolymer is a terpolymer of acrylamide, 2-dimethylammoniumethyl (meth)acrylate quaternized with methyl chloride (ADAME-Q) and 3-dimethylammoniumpropyl(meth)acrylamide quaternized with methyl chloride (DIMAPA-Q). The cationic copolymer is formed from acrylamide and acrylamidopropyltrimethylammonium chloride, wherein the acrylamidopropyltrimethylammonium chloride has a charge density of from about 1.0 meq/g to about 3.0 meq/g.

The cationic copolymer is a trimethylammoniopropylmethacrylamide chloride-N-Acrylamide copolymer, which is also known as AM:MAPTAC. AM:MAPTAC may have a charge density of about 1.3 meq/g and a M.Wt. of about **1.1** million g/mol. The cationic copolymer is AM:ATPAC. AM:ATPAC may have a charge density of about 1.8 meq/g and a M.Wt. of about 1.1 million g/mol.

Cationic cellulose polymers

Suitable cationic cellulose polymers are salts of hydroxyethyl cellulose reacted with trimethyl ammonium substituted epoxide, referred to in the industry (CTFA) as Polyquaternium 10 and available from Dow/ Amerchol Corp. (Edison, N.J., USA) in their Polymer LR, **JR,** and KG series of polymers. Other suitable types of cationic cellulose include the polymeric quaternary ammonium salts of hydroxyethyl cellulose reacted with lauryl dimethyl ammonium-substituted epoxide referred to in the industry (CTFA) as Polyquaternium 24. These materials are available from Dow/ Amerchol Corp. under the tradename Polymer LM-200. Other suitable types of cationic cellulose include the polymeric quaternary ammonium salts of hydroxyethyl cellulose reacted with lauryl dimethyl ammonium-substituted epoxide and trimethyl ammonium substituted epoxide referred to in the industry (CTFA) as Polyquaternium 67. These materials are available from Dow/ Amerchol Corp. under the tradename SoftCAT Polymer SL-5, SoftCAT Polymer SL-30, Polymer SL-60, Polymer SL-100, Polymer SK-L, Polymer SK-M, Polymer SK-MH, and Polymer SK-H.

Extensional Aids

The fibrous elements can contain extensional aids. Non-limiting examples of extensional aids can include polymers, other extensional aids, and combinations thereof.

In one example, the extensional aids have a weight average molecular weight of at least about 500,000 Da. The weight average molecular weight of the extensional aid is from about 500,000 Da to about 25,000,000 Da, alternatively from about 800,000 Da to about 22,000,000 Da, alternatively from about 1,000,000 Da to about 20,000,000 Da, and alternativley from about 2,000,000 Da to about 15,000,000 Da. The relatively high weight average molecular weight

extensional aids can be preferred in some examples of the invention due to the ability to increase extensional melt viscosity and reducing melt fracture.

The extensional aid, when used in a meltblowing process, can be added to the composition of the present invention in an amount effective to visibly reduce the melt fracture and capillary breakage of fibers during the spinning process such that substantially continuous fibers having relatively consistent diameter can be melt spun. Regardless of the process employed to produce fibrous elements and/or particles, the extensional aids, when used, can be present from about 0.001% to about 10%, by weight on a dry fibrous element basis and/or dry fibrous article basis, in one example, and in another example from about 0.005 to about 5%, by weight on a dry fibrous element basis and/or dry fibrous article basis, in yet another example from about 0.01 to about 1%, by weight on a dry fibrous element basis and/or dry fibrous article basis, and in another example from about 0.05% to about 0.5%, by weight on a dry fibrous element basis and/or dry fibrous article basis.

Non-limiting examples of polymers that can be used as extensional aids can include alginates, carrageenans, pectin, chitin, guar gum, xanthum gum, agar, gum arabic, karaya gum, tragacanth gum, locust bean gum, alkylcellulose, hydroxyalkylcellulose, carboxyalkylcellulose, and mixtures thereof.

Nonlimiting examples of other extensional aids can include modified and unmodified polyacrylamide, polyacrylic acid, polymethacrylic acid, polyvinyl alcohol, polyvinylacetate, polyvinylpyrrolidone, polyethylene vinyl acetate, polyethyleneimine, polyamides, polyalkylene oxides including polyethylene oxide, polypropylene oxide, polyethylenepropylene oxide, and mixtures thereof.

Optional Ingredients

The article can optionally comprise from about 1 wt.% to about 25 wt.% plasticizer, in one embodiment from about 3 wt. % to about 20 wt.% plasticizer, in one embodiment from about 5 wt.% to about 15 wt.% plasticizer.

When present in the articles, non-limiting examples of suitable plasticizing agents include polyols, copolyols, polycarboxylic acids, polyesters and dimethicone copolyols.

Examples of useful polyols include, but are not limited to, glycerin, diglycerin, propylene glycol, ethylene glycol, butylene glycol, pentylene glycol, cyclohexane dimethanol, hexane diol, polyethylene glycol (200-600), sugar alcohols such as sorbitol, manitol, lactitol, isosorbide, glucamine, N-methylglucamine and other mono- and polyhydric relatively low weight average molecular weight alcohols (e.g., $C_2$-$C_8$ alcohols); mono di- and oligo-saccharides such as fructose, glucose, sucrose, maltose, lactose, and high fructose corn syrup solids and ascorbic acid.

Examples of polycarboxylic acids include, but are not limited to citric acid, maleic acid, succinic acid, polyacrylic acid, and polymaleic acid.

Examples of suitable polyesters include, but are not limited to, glycerol triacetate, acetylated-monoglyceride, diethyl phthalate, triethyl citrate, tributyl citrate, acetyl triethyl citrate, acetyl tributyl citrate.

Examples of suitable dimethicone copolyols include, but are not limited to, PEG-12 dimethicone, PEG/PPG-18/18 dimethicone, and PPG-12 dimethicone.

Other suitable plasticizers include, but are not limited to, alkyl and allyl phthalates; napthalates; lactates (e.g., sodium, ammonium and potassium salts); sorbeth-30; urea; lactic acid; sodium pyrrolidone carboxylic acid (PCA); sodium hyraluronate or hyaluronic acid; soluble collagen; modified protein; monosodium L-glutamate; alpha & beta hydroxyl acids such as glycolic acid, lactic acid, citric acid, maleic acid and salicylic acid; glyceryl polymethacrylate; polymeric plasticizers such as polyquaterniums; proteins and amino acids such as glutamic acid, aspartic acid, and lysine; hydrogen starch hydrolysates; other relatively low weight average molecular weight esters (e.g., esters of $C_2$-$C_{10}$ alcohols and acids); and any other water soluble plasticizer known to one skilled in the art of the foods and plastics industries; and mixtures thereof. EP 0283165 B1 discloses suitable plasticizers, including glycerol derivatives such as propoxylated glycerol.

The article may comprise other optional ingredients that are known for use or otherwise useful in compositions, provided that such optional materials are compatible with the selected essential materials described herein, or do not otherwise unduly impair product performance.

Such optional ingredients are most typically those materials approved for use in cosmetics and that are described in reference books such as the CTFA Cosmetic Ingredient Handbook, Second Edition, The Cosmetic, Toiletries, and Fragrance Association, Inc. 1992.

Emulsifiers suitable as an optional ingredient herein include mono- and di-glycerides, fatty alcohols, polyglycerol esters, propylene glycol esters, sorbitan esters and other emulsifiers known or otherwise commonly used to stabilized air interfaces, as for example those used during preparation of aerated foodstuffs such as cakes and other baked goods and confectionary products, or the stabilization of cosmetics such as hair mousses.

Further non-limiting examples of such optional ingredients include preservatives, perfumes or fragrances, coloring agents or dyes, conditioning agents, hair bleaching agents, thickeners, moisturizers, emollients, pharmaceutical actives, vitamins or nutrients, sunscreens, deodorants, sensates, plant extracts, nutrients, astringents, cosmetic particles, absorbent particles, adhesive particles, hair fixatives, fibers, reactive agents, skin lightening agents, skin tanning agents,

antidandruff agents, perfumes, exfoliating agents, acids, bases, humectants, enzymes, suspending agents, hair colorants, hair perming agents, pigment particles, anti-acne agents, anti-microbial agents, sunscreens, tanning agents, exfoliation particles, hair growth or restorer agents, insect repellents, shaving lotion agents, co-solvents or other additional solvents, and similar other materials. Further non-limiting examples of optional ingredients include encapsulated perfumes, such as by β-cyclodetrins, polymer microcapsules, starch encapsulated accords and combinations thereof.

Suitable conditioning agents can optionally be added to the articles and can include high melting point fatty materials and silicone conditioning agents. Suitable materials are discussed in US 2008/0019935, US 2008/0242584 and US 2006/0217288.

Methods of Use

The compositions described herein may be used for cleaning, conditioning, and/or treating hair, hair follicles, and/or skin including the scalp. The method for treating these consumer substrates may comprise the steps of: a) applying an effective amount of the article to the hand, b) wetting the article with water to dissolve the solid, c) applying the dissolved material to the target consumer substrate to form a lather to clean and optionally condition, and d) rinsing the diluted treatment composition from the consumer substrate. These steps can be repeated as many times as desired to achieve the desired cleansing and or treatment benefit.

A method useful for providing a benefit to hair, hair follicles, and/or skin including the scalp, includes the step of applying a composition according to the first embodiment to these target consumer substrates in need of regulating.

Alternatively, a useful method for regulating the condition of hair, hair follicles, skin, and/or skin including the scalp, includes the step of applying one or more compositions described herein to these target consumer substrates in need of regulation.

The amount of the composition applied, the frequency of application and the period of use will vary widely depending upon the purpose of application, the level of components of a given composition and the level of regulation desired. For example, when the composition is applied for whole body or hair treatment, effective amounts generally range from about 0.5 grams to about 10 grams, alternatively from about 1.0 grams to about 5 grams, and alternatively from about 1.5 grams to about 3 grams.

Product Types and Articles of Commerce

Non-limiting examples of products that utilize the fibrous article include hand cleansing substrates, hair shampoo, hair conditioner or other hair treatment substrates, body cleansing substrates, shaving preparation substrates, personal care substrates containing pharmaceutical or other skin care active, moisturizing substrates, sunscreen substrates, chronic skin benefit agent substrates (e.g., vitamin-containing substrates, alpha-hydroxy acid-containing substrates, etc.), deodorizing substrates, fragrance-containing substrates, and so forth.

Described herein is an article of commerce comprising one or more fibrous articles described herein, and a communication directing a consumer to dissolve the article and apply the dissolved mixture to hair, hair follicles, and/or skin including the scalp, to achieve a benefit to the target consumer substrate, a rapidly lathering foam, a rapidly rinsing foam, a clean rinsing foam, a conditioning treatment and combinations thereof. The communication may be printed material attached directly or indirectly to packaging that contains the fibrous article or on the fibrous article itself. Alternatively, the communication may be an electronic or a broadcast message that is associated with the article of manufacture. Alternatively, the communication may describe at least one possible use, capability, distinguishing feature and/or property of the article of manufacture.

Exposure to Triggering Condition

The shampoo ingredients, including the surfactant and optionally the cationic polymer, may be released from the fibrous element and/or fibrous article when the fibrous element and/or fibrous article is exposed to a triggering condition. In one example, one or more active agents may be released from the fibrous element and/or fibrous article or a part thereof when the fibrous element and/or fibrous article or the part thereof loses its identity, in other words, loses its physical structure. For example, a fibrous element and/or fibrous article loses its physical structure when the polymeric structurant dissolves, melts or undergoes some other transformative step such that its structure is lost. In one example, the one or more active agents are released from the fibrous element and/or fibrous article when the fibrous element's and/or fibrous article's morphology changes.

In another example, one or more active agents may be released from the fibrous element and/or fibrous article or a part thereof when the fibrous element and/or fibrous article or the part thereof alters its identity, in other words, alters its physical structure rather than loses its physical structure. For example, a fibrous element and/or fibrous article alters its physical structure when the polymeric structurant swells, shrinks, lengthens, and/or shortens, but retains its filament-forming properties.

In another example, one or more active agents may be released from the fibrous element and/or fibrous article with its morphology not changing (not losing or altering its physical structure).

In one example, the fibrous element and/or fibrous article may release an active agent upon the fibrous element and/or fibrous article being exposed to a triggering condition that results in the release of the active agent, such as by causing the fibrous element and/or fibrous article to lose or alter its identity as discussed above. Non-limiting examples of triggering conditions include exposing the fibrous element and/or fibrous article to solvent, a polar solvent, such as alcohol and/or water, and/or a non-polar solvent, which may be sequential, depending upon whether the filament-forming composition comprises a polar solvent-soluble material and/or a non-polar solvent-soluble material; exposing the fibrous element and/or particle and/or fibrous article to heat, such as to a temperature of greater than 75°F and/or greater than 100°F and/or greater than 150°F and/or greater than 200°F and/or greater than 212°F; exposing the fibrous element and/or particle and/or fibrous article to cold, such as to a temperature of less than 40°F and/or less than 32°F and/or less than 0°F; exposing the fibrous element and/or fibrous article to a force, such as a stretching force applied by a consumer using the fibrous element and/or fibrous article; and/or exposing the fibrous element and/or fibrous article to a chemical reaction; exposing the fibrous element and/or fibrous article to a condition that results in a phase change; exposing the fibrous element and/or fibrous article to a pH change and/or a pressure change and/or temperature change; exposing the fibrous element and/or fibrous article to one or more chemicals that result in the fibrous element and/or fibrous article releasing one or more of its active agents; exposing the fibrous element and/or particle and/or fibrous article to ultrasonics; exposing the fibrous element and/or fibrous article to light and/or certain wavelengths; exposing the fibrous element and/or fibrous article to a different ionic strength; and/or exposing the fibrous element and/or fibrous article to an active agent released from another fibrous element and/or fibrous article.

In one example, one or more active agents may be released from the fibrous elements of the present invention when a fibrous article product comprising the fibrous elements is subjected to a triggering step such as forming a wash liquor by contacting the fibrous article product with water.

Method for Making Fibrous Elements and Articles

The fibrous elements of the present invention may be made by any suitable process. A non-limiting example of a suitable process for making the fibrous elements is described below.

In one example, as shown in FIGS. 5 and 6 a method 46 for making a fibrous element 32 according to the present invention comprises the steps of:

a. providing a filament-forming composition 48 comprising one or polymeric structurants, and optionally one or more other ingredients including high melting point fatty materials and/or one or more surfactants, wherein the filament-forming composition can comprise a pH of greater than about 5.5, alternatively greater than about 5.8, alternatively greater than 6.0; and

b. spinning the filament-forming composition 48, such as via a spinning die 50, into one or more fibrous elements 32, such as filaments, comprising the one or more polymeric structurants and optionally, the one or more other ingredients. The one or more other ingredients may be releasable from the fibrous element when exposed to conditions of intended use. The total level of the one or more polymeric structurants present in the fibrous element 32, may be less than 80% and/or less than 70% and/or less than 65% and/or 50% or less by weight on a dry fibrous element basis and/or dry fibrous article basis and the total level of the one or more active agents, when present in the fibrous element may be greater than 20% and/or greater than 35% and/or 50% or greater 65% or greater and/or 80% or greater by weight on a dry fibrous element basis and/or dry fibrous article basis.

As shown in FIG. 6, the spinning die 50 may comprise a plurality of fibrous element-forming holes 52 that include a melt capillary 54 encircled by a concentric attenuation fluid hole 56 through which a fluid, such as air, passes to facilitate attenuation of the filament-forming composition 48 into a fibrous element 32 as it exits the fibrous element-forming hole 52. It was found that if the filament forming composition had a pH of greater than about 5.5, better filaments can form after drying.

In one example, during the method for making fibrous elements, any volatile solvent, such as water, present in the filament-forming composition 48 is removed, such as by drying, as the fibrous element 32 is formed. In one example, greater than 30% and/or greater than 40% and/or greater than 50% and/or greater than 60% of the weight of the filament-forming composition's volatile solvent, such as water, is removed during the spinning step, such as by drying the fibrous element being produced.

It was found that during the spinning step, the inventive examples in Table 1, Table 3, and Table 4, below, can be sensitive to excessive heat exposure during the method for making fibrous elements. For example, if the fibrous elements are exposed to excessive heat for too long the fibrous elements can have active degradation and/or color change and/or

odor change. However, the temperature needs to be high enough so the solvent can evaporate within an acceptable time period. In one example, when the fibrous element exits the fibrous element-forming hole 52, they are collected on a belt above a vacuum source called the forming zone. The fibrous elements can remain on the forming zone for the following times and temperatures: from about 150 °F (65.6 °C) to about 160 °F (71.1 °C) for about 50 to about 60 seconds and/or from about 170 °F (65.6 °C) to about 180 °F (82.2 °C) for about 30 to about 40 seconds and/or from about 200 °F (93.3 °C) to about 215 °F (101.7 °C) for about 5 to about 20 seconds.

In one example, to enable the balance of solvent evaporation, dwell time, and heat exposure it is apparent that melt spinning temperature could be from about 70 °F to about 95 °F while enabling drying with heat such as about 340 °F (171.1 °C) to about 350 °F (176.7 °C) for about 50 to about 60 seconds or from about 390 °F (198.9 °C) to about 400 °F (204 °C) for about 30 to about 40 seconds or 415 °F (212.8 °C) to 470 °F (243.3 °C) for about 5 to about 20 seconds.

The filament-forming composition may comprise any suitable total level of polymeric structurant and any suitable level of active agents so long as the fibrous element produced from the filament-forming composition comprises a total level of polymeric structurant in the fibrous element of from about 5% to 50% or less by weight on a dry fibrous element basis and/or dry fibrous article basis and a total level of active agents in the fibrous element of from 50% to about 95% by weight on a dry fibrous element basis and/or dry fibrous article basis.

In one example, the filament-forming composition may comprise any suitable total level of polymeric structurant and any suitable level of active agents so long as the fibrous element produced from the filament-forming composition comprises a total level of polymeric structurant in the fibrous element and/or particle of from about 5% to 50% or less by weight on a dry fibrous element basis and/or dry fibrous article basis and a total level of active agents in the fibrous element and/or particle of from 50% to about 95% by weight on a dry fibrous element basis and/or dry fibrous article basis, wherein the weight ratio of polymeric structurant to total level of surfactant and/or high melting point fatty material is 1 or less.

In one example, the filament-forming composition comprises from about 1% and/or from about 5% and/or from about 10% to about 50% and/or to about 40% and/or to about 30% and/or to about 20% by weight of the filament-forming composition of polymeric structurant; from about 1% and/or from about 5% and/or from about 10% to about 50% and/or to about 40% and/or to about 30% and/or to about 20% by weight of the filament-forming composition of active agents; and from about 20% and/or from about 25% and/or from about 30% and/or from about 40% and/or to about 80% and/or to about 70% and/or to about 60% and/or to about 50% by weight of the filament-forming composition of a volatile solvent, such as water. The filament-forming composition may comprise minor amounts of other active agents, such as less than 10% and/or less than 5% and/or less than 3% and/or less than 1% by weight of the filament-forming composition of plasticizers, pH adjusting agents, and other active agents.

The filament-forming composition is spun into one or more fibrous elements and/or particles by any suitable spinning process, such as meltblowing, spunbonding, electro-spinning, and/or rotary spinning. In one example, the filament-forming composition is spun into a plurality of fibrous elements and/or particles by meltblowing. For example, the filament-forming composition may be pumped from a tank to a meltblown spinnerette. Upon exiting one or more of the filament-forming holes in the spinnerette, the filament-forming composition is attenuated with air to create one or more fibrous elements and/or particles. The fibrous elements and/or particles may then be dried to remove any remaining solvent used for spinning, such as the water.

The fibrous elements and/or particles of the present invention may be collected on a belt, such as a patterned belt to form a fibrous article comprising the fibrous elements and/or particles.

## TEST METHODS

Unless otherwise specified, all tests described herein including those described under the Definitions section and the following test methods are conducted on samples that have been conditioned in a conditioned room at a temperature of 22 °C ± 2 °C and a relative humidity of 42% ± 4% for a minimum of 2 hours prior to the test. The samples tested are "usable units." "Usable units" as used herein means sheets, flats from roll stock, pre-converted flats, and/or single or multiply products. All tests are conducted under the same environmental conditions and in such conditioned room. Do not test samples that have defects such as wrinkles, tears, holes, and like. Samples conditioned as described herein are considered dry samples (such as "dry filaments") for testing purposes. All instruments are calibrated according to manufacturer's specifications.

### Diameter Test Method

The diameter of a discrete fibrous element or a fibrous element within a fibrous article is determined by using a Scanning Electron Microscope (SEM) or an Optical Microscope and an image analysis software. A magnification of 200 to 10,000 times is chosen such that the fibrous elements are suitably enlarged for measurement. When using the SEM, the samples are sputtered with gold or a palladium compound to avoid electric charging and vibrations of the fibrous element in the electron beam. A manual procedure for determining the fibrous element diameters is used from the image (on monitor

screen) taken with the SEM or the optical microscope. Using a mouse and a cursor tool, the edge of a randomly selected fibrous element is sought and then measured across its width (i.e., perpendicular to fibrous element direction at that point) to the other edge of the fibrous element. A scaled and calibrated image analysis tool provides the scaling to get actual reading in μm. For fibrous elements within a fibrous article, several fibrous elements are randomly selected across the sample of the fibrous article using the SEM or the optical microscope. At least two portions of the fibrous article are cut and tested in this manner. Altogether at least 100 such measurements are made and then all data are recorded for statistical analysis. The recorded data are used to calculate average (mean) of the fibrous element diameters, standard deviation of the fibrous element diameters, and median of the fibrous element diameters.

Another useful statistic is the calculation of the amount of the population of fibrous elements that is below a certain upper limit. To determine this statistic, the software is programmed to count how many results of the fibrous element diameters are below an upper limit and that count (divided by total number of data and multiplied by 100%) is reported in percent as percent below the upper limit, such as percent below 1 micrometer diameter or %-submicron, for example. We denote the measured diameter (in μm) of an individual circular fibrous element as di.

In the case that the fibrous elements have non-circular cross-sections, the measurement of the fibrous element diameter is determined as and set equal to the hydraulic diameter which is four times the cross-sectional area of the fibrous element divided by the perimeter of the cross-section of the fibrous element (outer perimeter in case of hollow fibrous elements). The number-average diameter, alternatively average diameter is calculated as:

$$d_{num} = \frac{\sum_{i=1}^{n} d_i}{n}$$

Fibrous Element Composition Test Method

In order to prepare fibrous elements for fibrous element composition measurement, the fibrous elements must be conditioned by removing any coating compositions and/or materials present on the external surfaces of the fibrous elements that are removable. An example of a method for doing so is washing the fibrous elements 3 times with a suitable solvent that will remove the external coating while leaving the fibrous elements unaltered. The fibrous elements are then air dried at 23°C ± 1.0°C until the fibrous elements comprise less than 10% moisture. A chemical analysis of the conditioned fibrous elements is then completed to determine the compositional make-up of the fibrous elements with respect to the filament-forming materials and the active agents and the level of the filament-forming materials and active agents present in the fibrous elements.

The compositional make-up of the fibrous elements with respect to the filament-forming material and the active agents can also be determined by completing a cross-section analysis using TOF-SIMs or SEM. Still another method for determining compositional make-up of the fibrous elements uses a fluorescent dye as a marker. In addition, as always, a manufacturer of fibrous elements should know the compositions of their fibrous elements.

Hand Dissolution Method

Materials Needed:

Fibrous articles to be tested: 3-5 fibrous articles (finished product samples) are tested so that an average of the number of strokes for each if the individual fibrous article samples is calculated and recorded as the Average Hand Dissolution value for the fibrous article. For this method, the entire consumer saleable or consumer use fibrous article is tested. If the entire consumer saleable or consumer use fibrous article has a footprint greater than 50 $cm^2$, then first cut the fibrous article to have a footprint of 50 $cm^2$.

Nitrile Gloves

10 cc syringe
Plastic Weigh boat (~3in x 3in)
100 mL Glass beaker

Water (City of Cincinnati Water or equivalent having the following properties: Total Hardness = 155mg/L as $CaCO_2$; Calcium content = 33.2 mg/L; Magnesium content = 17.5 mg/L; Phosphate content = 0.0462 mg/L). Water used is water 7 grains per gallon (gpg) hardness and 40°C +/- 5°C

Protocol:

- Add 80 mL of water to glass beaker.
- Heat water in beaker until water is at a temperature of 40°C +/- 5°C.
- Transfer 15 mL of the water from the beaker into the weigh boat via the syringe.
- Within 10 seconds of transferring the water to the weigh boat, place fibrous article sample in palm of gloved hand (hand in cupped position in non-dominant hand to hold fibrous article sample).
- Using dominant hand, add water quickly from the weigh boat to the fibrous article sample and allow to immediately wet for a period of 5-10 seconds.
- Rub with opposite dominant hand (also gloved) in 2 rapid circular strokes.
- Visually examine the fibrous article sample in hand after the 2 strokes. If fibrous article sample is completely dissolved, record number of strokes = 2 Dissolution Strokes. If not completely dissolved, rub remaining fibrous article sample for 2 more circular strokes (4 total) and observe degree of dissolution. If the fibrous article sample contains no solid pieces after the 2 additional strokes, record number of strokes = 4 Dissolution Strokes. If after the 4 strokes total, the fibrous article sample still contains solid pieces of un-dissolved fibrous article sample, continue rubbing remaining fibrous article sample in additional 2 circular strokes and check if there are any remaining solid pieces of fibrous article sample after each additional 2 strokes until fibrous article sample is completely dissolved or until reaching a total of 30 strokes, whichever comes first. Record the total number of strokes. Record 30 Dissolution Strokes even if solid fibrous article sample pieces remain after the maximum of 30 strokes.
- Repeat this process for each of the additional 4 fibrous article samples.
- Calculate the arithmetic mean of the recorded values of Dissolution Strokes for the 5 individual fibrous article samples and record as the Average Hand Dissolution Value for the fibrous article. The Average Hand Dissolution Value is reported to the nearest single Dissolution Stroke unit.

pH Test Method

The pH of a liquid composition is taken using a calibrated (according to the manufacturer's instructions) pH probe that is calibrated with 3 pH standards (4.0, 7.0, and 10.0). The pH is recorded to the tenth unit (i.e., 5.5).

Rheology Test Method

Discovery HR-3 Rheometer (TA Instruments®, Delaware, USA) equipped with a 40mm 2.002 degree cone plate, Peltier plate steel-106935 and a flat plate lower geometry equipped with a Peltier heating/cooling mechanism to control the temperature. Measurements are conducted by placing about 1 gram of the composition onto the lower plate geometry and lowering the upper plate geometry to a target gap of 60 microns, wiping away any excess of the respective fibrous element-forming composition to create an even surface flush with the edges of the upper and lower plate geometries. Data was collected at 25°C or 40°C as specified by performing an amplitude sweep with a oscillation Amplitude 1Hz, $6.366e^{-8}$ to $5.0e^{-4}$MPa to obtain G' and G" curves and a Flow Sweep 0.1 to 500 $S^{-1}$ to obtain shear viscosity. G', G" numbers in the data table were taken from the 1st data points at the lowest Oscillation strain R (%) (in the flat range) and shear viscosity at shear rate of 0.1 $s^{-1}$.

Thickness Method

Thickness of a fibrous article is measured by cutting 5 samples of a fibrous article sample such that each cut sample is larger in size than a load foot loading surface of a VIR Electronic Thickness Tester Model II available from Thwing-Albert Instrument Company, Philadelphia, PA. Typically, the load foot loading surface has a circular surface area of about 3.14 $in^2$. The sample is confined between a horizontal flat surface and the load foot loading surface. The load foot loading surface applies a confining pressure to the sample of 15.5 $g/cm^2$. The thickness of each sample is the resulting gap between the flat surface and the load foot loading surface. The thickness is calculated as the average thickness of the five samples. The result is reported in millimeters (mm).

Water Content Test Method

The water (moisture) content present in a fibrous element and/or particle and/or fibrous article is measured using the following Water Content Test Method. A fibrous element and/or particle and/or fibrous article or portion thereof ("sample") in the form of a pre-cut sheet is placed in a conditioned room at a temperature of 22 °C ± 2 °C and a relative humidity of 42% ± 4% for at least 24 hours prior to testing. Each fibrous article sample has an area of at least 4 square inches, but small enough in size to fit appropriately on the balance weighing plate. Under the temperature and humidity conditions mentioned above, using a balance with at least four decimal places, the weight of the sample is recorded every five minutes until a change of less than 0.5% of previous weight is detected during a 10-minute period. The final weight is recorded as the "equilibrium weight". Within 10 minutes, the samples are placed into the forced air oven on top of foil for 24 hours at 22 °C ± 2 °C and a relative humidity of 42% ± 4% for drying. After the 24 hours of drying, the sample is removed and weighed within 15 seconds. This weight is designated as the "dry weight" of the sample.
The water (moisture) content of the sample is calculated as follows:

$$\%\ \text{Water in sample} = 100\% \times \frac{(\text{Equilibrium weight of sample} - \text{Dry weight of sample})}{\text{Dry weight of sample}}$$

The % Water (moisture) in sample for 3 replicates is averaged to give the reported % Water (moisture) in sample. Report results to the nearest 0.1%.

EXAMPLES

The following are non-limiting examples of the shampoo compositions described herein.
All parts, percentages, and ratios herein are by weight unless otherwise specified. Some components may come from suppliers as dilute solutions. The amount stated reflects the weight percent of the added material, unless otherwise specified.
The melt compositions and fibrous articles described in Table 1 to Table 3, below, were made according to the methods described in this application.
Phase stability, in Table 1, below, was determined by visual detection of the Melt Composition. The Melt Composition was determined to be phase stable if by visual detection there is no phase separation, which includes precipitates, and the example appears homogeneous. As used herein, "visual detection" means that a human viewer can visually discern the quality of the example with the unaided eye (excepting standard corrective lenses adapted to compensate for near-sightedness, farsightedness, or astigmatism, or other corrected vision) in lighting at least equal to the illumination of a standard 100-watt incandescent white light bulb at a distance of approximately 1 foot (0.30 m).
Fiber spinnability, in Table 1, below, was determined by spinning the melt composition, according to the Method for Making Fibrous Elements and Articles described herein. If when spun the melt composition had the proper extensional rheology so it could extend to form filaments without breaking or retracting, then the melt was spinnable (see current invention spin ability criteria described in methods of making fibrous articles) described in forming section. If when spun, continuous filaments are not formed or filaments are broken before reaching the collecting belt, the melt is not spinnable. If the melt composition was stable and spinnable you can form filaments and webs according to the method described herein.

**Table 1: Melt Compositions**

| | Ex. A | Ex. B | Ex. 1 | Ex. 2 | Ex. 3 | Ex. 4 |
|---|---|---|---|---|---|---|
| Polyvinyl alcohol[1] (PVA) | 12.0 | 13.3 | 11.49 | 13.0 | 13.0 | 13.0 |
| Disodium cocoyl glutamate[2A] | 31.11 (50% solids) | 34.55 (50% solids) | 38.85 (50% solids) | 46.0 (50% solids) | 14.0 (50% solids) | 44.0 (50% solids) |
| Sodium cocyl alaninate[2B] | - | - | - | 16.7 (30% solids) | 57.25 (30% solids) | - |
| Sodium cocoyl isethionate[3] | 4.0 | 4.44 | 5.25 | - | - | - |

(continued)

| | Ex. A | Ex. B | Ex. 1 | Ex. 2 | Ex. 3 | Ex. 4 |
|---|---|---|---|---|---|---|
| LAPB[4] | 6.86 (35% solids) | 7.62 (35% solids) | 9.0 (35% solids) | - | - | - |
| Cocamidopropyl betaine[11] | - | - | - | - | - | 8.57 |
| EDTA[5] | 0.16 | 0.16 | 0.16 | 0.16 | 0.10 | - |
| Citric acid[6] | 1.28 | 1.40 | - | - | - | - |
| Lactic Acid[7] | - | - | 2.0 (90% solids) | 2.0 (90% solids) | 1.44 (90% solids) | 3.0 |
| Polyquaternium -6[8] | 0.50 (40% solids) | 0.56 (40% solids) | 0.50 (40% solids) | 0.50 (40% solids) | 0.50 (40% solids) | 0.50 (40% solids) |
| Polyquaternium -10[9] | 0.20 | 0.22 | 0.20 | - | 0.20 | - |
| Water | Q.S. | Q.S. | Q.S. | Q.S. | Q.S. | Q.S. |
| Acid Type | Citric | Citric | Lactic | Lactic | Lactic | Lactic |
| Total % Melt Solids Content | 36.0 | 40.0 | 41.7 | 43.0 | 39.0 | 40.0 |
| Total surfactant to PVA ratio | 1.83 | 1.83 | 2.42 | 2.15 | 1.86 | 1.92 |
| G' (Pa at 25°C) | 55.7 | 17.2 | 165 | - | - | - |
| G" (Pa at 25°C) | 165.7 | 88.8 | 337 | - | - | - |
| Tan delta at 25°C (G"/G') | 3.0 | 5.2 | 2.0 | - | - | - |
| G' (Pa at 40°C) | 14.8 | - | 50 | 33.0 | 38.1 | 26.0 |
| G" (Pa at 40°C) | 68.2 | - | 152 | 138 | 119 | 86 |
| Tan delta (40°C) | 4.6 | - | 3.0 | 4.2 | 3.1 | 3.3 |
| Phase stable at 25°C? | Yes | Yes | Yes | Yes | Yes | Yes |
| Phase stable at 40°C? | No | No | Yes | Yes | Yes | Yes |
| Able to spin at 25°C | Yes | No | No | - | - | - |
| Able to spin at 40°C | No | No | Yes | Yes | Yes | Yes |

In Table 1, Example A can be spun into fibrous elements at 25°C. However, it has a total melt solids content of 36% and it can be desirable to increase this level to make the process more efficient. In addition, the composition cannot be spun into fibrous elements at 40°C, which is a more efficient temperature. Example B has 40% total melt solids, however, it cannot be spun into fibrous elements at 25°C or 40°C, in part because the composition is not phase stable at 40°C and does not have the correct rheology at 25°C and also because the composition does not have sufficient rheology (G') at either temperature. Examples 1-4 can be preferred, because it is more efficient to make these fibrous elements as compared to making fibrous elements of Example A, while still being able to be spun at 40°C, unlike Example B. Example 1 contains 41.7% total melt solids and is phase stable and spinnable at 40°C. However, at 25°C, the melt of Example 1 has a G' > 70 and tan delta < 2.8. It cannot be spun well into fibers at 25°C. But at 40°C, it has a G' of 50 and tan delta of 3.0 and it can be spun into fibers. Example 2 contains 43% solids and is phase stable and spinnable at 40°C. Example 3 contains 39% solids and is phase stable and spinnable at 40°C. Example 4 contains 40% solids and is phase stable and spinnable at 40°C. The higher total % melt solids in Examples 1-4 not only have a higher total % melt solids, as compared to Example A, but Examples 1-4 also have a higher ratio of active surfactant to PVA ratio, as compared to Example A and Example B. Thus, Examples 1-4 deliver more active at the same weight of the dry fibrous article.

The melt compositions of Example A and Example 1 in Table 1 are spun into fibrous elements and then made into

fibrous shampoo articles. Table 2, below, shows Ex. A' (made from the Ex. A melt that was spun into fibrous elements at 25°C) and Ex. 1' (made from the Ex. 1 melt that was spun into fibrous elements at 40°C). A fibrous shampoo article from Example B is not shown, because the melt does not make fibrous elements that can be made into fibrous articles.

**Table 2: Fibrous Shampoo Articles**

| | Ex. A' (spun at 25°C) | Ex. 1' (spun at 40°C) |
|---|---|---|
| Polyvinyl alcohol[1] | 29.0 | 23.2 |
| Disodium cocoyl Glutamate[2A] | 36.37 | 39.23 |
| Sodium cocoyl isethionate[3] | 9.65 | 10.60 |
| LAPB[4] | 5.81 | 6.36 |
| EDTA[5] | 0.43 | 0.32 |
| Citric acid[6] | 3.2 | - |
| Lactic Acid[7] | - | 3.63 |
| Polyquaternium-6[8] | 0.51 | 0.40 |
| Polyquaternium-10[9] | 0.51 | 0.40 |
| Perfume | 6.78 | 7.51 |
| Silicone[10] | 4.74 | 5.26 |
| Moisture | 3.1 | 3.1 |

**Table 3: Melt Composition**

| | Ex. 5 |
|---|---|
| Polyvinyl alcohol[1] | 38.34 (pre-dissolved 30% solids) |
| Disodium cocoyl Glutamate[2A] | 38.75 (50% solids) |
| Sodium cocoyl isethionate[3] | 5.24 |
| LAPB[4] | 8.99 (35% solids) |
| EDTA[5] | 0.16 |
| Lactic Acid[7] | 2.0 (90% solids) |
| Polyquaternium-6[8] | 0.50 (40% solids) |
| Polyquaternium-10[9] | 0.20 |
| Water | Q.S. |

Suppliers for raw materials for the Examples in Table 1 to Table 3:

1. Poval 32-80, Poval 3-80 (50:50 blend) from Kuraray®
2A. Eversoft™ UCS-50SG from Sino Lion™
2B. Eversoft™ ACS-30 from Sino Lion™
3. Jordapon® CI Prill from BASF®
4. Mackam® DAB ULS from Solvay®
5. Versene™ 220 from Dow®
6. Citric acid from ADM™
7. Lactic Acid available as PURAC® HiPure 90 from Corbion®
8. Polyquaternium-6, PolyDADMAC, MW of 150,000, CD of 6.2, trade name: Mirapol® 100s, 31.5% active, 40% solids from Solvay®
9. Polyquaternium-10, UCARE™ Polymer JR-30M from Amerchol®, MW of 2,000,000, CD of 1.25
10. Silicone: Y-14945, amodimethicone, from Momentive®
11. TEGO BETAIN CK PH 12 from Evonik®

**Table 4: Melt Compositions (Ex. A from Table 1 and Ex. 5 from Table 3) with and without an additive**

| Example | pH | G' (Pa at 25°C) | G" (Pa at 25°C) | Shear Viscosity (Pa.s at 25°C) |
|---|---|---|---|---|
| Ex. A | 5.9 | 55.7 | 165.7 | 44.2 |
| Ex. A (99.88%) + 0.12% Citric Acid | 5.7 | 34.7 | 133 | 32.9 |
| Ex. A (99.78%) + 0.22% Citric Acid | 5.5 | 17.2 | 88.8 | 18.3 |
| Ex. 5 | Not measured | 205 | 360 | 340 |
| Ex. 5 (96%) + 4% sodium citrate | Not measured | 70 | 140 | 150 |

The melt compositions in Table 4 show how an additive can lower rheology and can be used to approximate how a hydrated article could feel in a user's hand.

Example A, without an additive, has a rheology that can be spun into fibrous elements. However, when a fibrous article made from this melt is hydrated, it is expected to feel sticky, stringy, and/or gooey in a user's hands that some consumers can find bothersome.

When 0.12% citric acid is added to the Example A melt and when 0.22% citric acid is added to the Example A melt the G', G", and shear viscosity is reduced, as compared to Example A without the citric acid additive. This indicates that if Example A was made into a fibrous elements and then citric acid was added to the fibrous elements and/or a fibrous article as a coating, the rheology could be preferable to some consumers because it could feel less sticky and it could be easier to disperse throughout a user's hair. Table 1 and the accompanying text

Example 5, without an additive, is expected to feel is expected to feel sticky, stringy, and/or gooey in a user's hands after it is hydrated, based on the rheology of the melt composition. Some users can find this rheology bothersome. This indicates that a fibrous article made from Example 5 that includes a sodium citrate additive may have a preferable in-hand feel that is preferred by some consumers, as compared to Example 5, since the sticky rheology is significantly reduced in Example 5 with an additive.

Table 5, shows the data from a sensory panel that rated the fibrous article Ex. 1' from Table 2 and the fibrous article Ex. 1' from Table 2 with 0.2 g of an additive. The additive contains citric acid, sodium bicarbonate, and polyvinylpyrrolidone, and zeolite A. For Ex. 1' with the additive, the additive particles were dropped onto the web via a hopper during fiber spinning.

The sensory panel was performed as follows:

- A up was filled with warm tap water from Mason, Ohio and this was used to fill a syringe to 7 mL and it was set aside.
- The panelist washed their hands with one pump of Cetaphil® Gentle Skin Cleanser, and rinsed with the tap water until the later was completely gone.
- The panelist's hands were then rewet under the faucet with the tap water and shook hands once to remove excess water
- A fibrous shampoo article was placed in the palm of the panelist's non-dominant hand that was held in a cupped position.
- Next, 7 mL of water was quickly and evenly dispensed from the syringe to the article starting at the edges and working its way into the middle.
- The article hydrated for two seconds and then rubbed with the panelist's dominant hand in two rapid rotations. The panelist looked at the product in hand and noted the look and feel.
- Then, the panelist rubbed hands together for ten more rotations (to reach 10 total rotations). The panelist looked at the product in hand and noted the look and feel.
- Finally, the scores were recorded and the mean at each time period was determined.

**Table 5: Sensory Panel Evaluation of In-Hand Rheology (Mean Panelist Score)**

| Sensory attribute | Ex. 1' | Ex. 1' + 0.2 g additive* per article |
|---|---|---|
| Product feel and texture after second rotation (thin to thick) (0-10) | 6.89 (0.59) | 3.07 (0.43) |
| Product feel and texture after second rotation (slippery to sticky) (0-10) | 6.96 (0.41) | 4.04 (0.46) |
| Product feel and texture after tenth rotation (thin to thick) (0-10) | 7.11 (0.54) | 2.89 (0.60) |

(continued)

| Sensory attribute | Ex. 1’ | Ex. 1’ + 0.2 g additive* per article |
|---|---|---|
| Product feel and texture after tenth rotation (slippery to sticky) (0-10) | 6.93 (0.53) | 4.04 (0.51) |
| *Additive comprises 52.6% citric acid, 32.2% sodium bicarbonate, 1.3% polyvinylperolidone and 14.0% Zeolite A. | | |

Table 5 shows that Ex. 1’ with the additive coating feels thinner and more slippery than Ex. 1’ that lacks a coating. Ex, 1’ could be preferred by some consumers because they may think that it feels more like traditional liquid shampoo products.

## Claims

**1.** A dissolvable solid fibrous shampoo article comprising a plurality of fibrous elements comprising:

a. from 1% to 50%, preferably from 20% to 70%, more preferably from 35% to 60%, by weight on a dry article basis, of a polymeric structurant, wherein the polymeric structurant is selected from carboxymethyl cellulose, starch, polyvinyl alcohol, or combinations thereof, wherein the total amount of polymeric structurant ranges from 1% to 50% by weight on a dry article basis;
b. from 20% to 70%, preferably from 40% to 70%, more preferably from 45% to 65%, by weight on a dry article basis, of a surfactant system comprising:

i. from 35% to 90%, preferably from 45% to 85%, more preferably from 50% to 80%, by weight of the surfactant system on a dry article basis, of a primary anionic surfactant, wherein the primary anionic surfactant comprises a glutamate surfactant selected from the group consisting of sodium cocoyl glutamate, disodium cocoyl glutamate, potassium cocoyl glutamate, dipotassium cocoyl glutamate, ammonium cocoyl glutamate, diammonium cocoyl glutamate, TEA-cocoyl glutamate, or combinations thereof or an alaninate surfactant selected from sodium cocoyl alaninate, sodium lauroyl alaninate, sodium N-dodecanoyl-1-alaninate, and combinations thereof, wherein the total amount of primary anionic surfactant ranges from 35% to 90% by weight of a dry article basis; and
ii. from 10% to 65%, preferably from 15% to 55%, more preferably from 23% to 50%, by weight of the surfactant system on a dry article basis, of a co-surfactant;
wherein the surfactant system comprises less than 0.1% of sulfate-based surfactants by total weight of the fibrous elements;

c. from 0.5% to 5%, preferably from 0.75% to 3.7%, more preferably from 1% to 3%, by weight on a dry article basis, of a pH adjuster, wherein the pH adjuster consists of a monoprotic organic acid; and wherein the monoprotic organic acid is dispersed throughout the fibrous elements, wherein the monoprotic organic acid is selected from the group consisting of lactic acid, acetic acid, glycolic acid, glyceric acid, and combinations thereof, wherein the total amount of pH adjuster ranges from 0.5% to 5% by weight of a dry article basis;
d. wherein the fibrous elements comprises less than 0.1% of citric acid by total weight of the fibrous elements;
e. wherein the plurality of fibrous elements are intertangled or otherwise associated with one another to form the fibrous article.

**2.** The article of claim1, wherein the monoprotic organic acid comprises lactic acid.

**3.** The article according to any preceding claims, wherein the fibrous elements are homogeneous.

**4.** The article according to any preceding claims, wherein the article further comprises a cationic polymer selected from the group consisting of Polyquaternium-6, Polyquaternium-10, cationic guars, and combinations thereof.

**5.** The article according to any preceding claims, wherein the article comprises a hand dissolution value of less than 154 strokes, preferably less than 12 strokes, more preferably less than 10 strokes according to the Hand Dissolution Test Method as disclosed herein.

**6.** The article according to any preceding claims, wherein the article further comprises a coating comprising an additive

selected from the group consisting of hydrotropes, diprotic and triprotic organic acids and salts thereof, and combinations thereof.

**7.** The article of claim 6, wherein the diprotic and triprotic organic acids are selected from the group consisting of citric acid, oxalic acid, malonic acid, tartronic acid, fumaric acid, maleic acid, malic acid, tartaric acid, and salts thereof, and combinations thereof.

**8.** The article of claim 7, wherein the diprotic and triprotic organic acid comprises citric acid and salts thereof.

**9.** The article of claim 6, wherein the hydrotropes are selected from the group consisting of sodium xylene sulfonate, urea, sodium toluenesulfonate, and combinations thereof.

**10.** The article of according to any preceding claims, wherein the article comprises a G' of from 5 Pa to 150 Pa at 25°C, preferably from 10 Pa to 100 Pa at 25°C, more preferably from 12 Pa to 75 Pa at 25°C, according to the Rheology Test Method as disclosed herein, when the article is hydrated with 7 mL of tap water per 2.5 grams of article.

**11.** Use of the article according to any preceding claims for providing a shampoo composition that is smooth and creamy when the article is hydrated.

**Patentansprüche**

**1.** Auflösbarer fester faseriger Shampooartikel, umfassend eine Vielzahl von faserigen Elementen, umfassend:

a. zu 1 Gew.-% bis 50 Gew.-%, vorzugsweise zu 20 Gew.-% bis 70 Gew.-%, mehr bevorzugt zu 35 Gew.-% bis 60 Gew.-%, auf Basis eines trockenen Artikels, eines polymeren Strukturmittels, wobei das polymere Strukturmittel ausgewählt ist aus Carboxymethylcellulose, Stärke, Polyvinylalkohol oder Kombinationen davon, wobei die Gesamtmenge des polymeren Strukturmittels in einem Bereich von 1 Gew.-% bis 50 Gew.-%, auf Basis eines trockenen Artikels, liegt;
b. zu 20 Gew.-% bis 70 Gew.-%, vorzugsweise zu 40 Gew.-% bis 70 Gew.-%, mehr bevorzugt zu 45 Gew.-% bis 65 Gew.-%, auf Basis eines trockenen Artikels, ein Tensidsystem, umfassend:

i. zu 35 Gew.-% bis 90 Gew.-%, vorzugsweise zu 45 Gew.-% bis 85 Gew.-%, mehr bevorzugt zu 50 Gew.-% bis 80 Gew.-% des Tensidsystems, auf Basis eines trockenen Artikels, ein primäres anionisches Tensid, wobei das primäre anionische Tensid ein Glutamattensid umfasst, ausgewählt aus der Gruppe, bestehend aus Natriumcocoylglutamat, Dinatriumcocoylglutamat, Kaliumcocoylglutamat, Dikaliumcocoylglutamat, Ammoniumcocoylglutamat, Diammoniumcocoylglutamat, TEA-Cocoylglutamat oder Kombinationen davon oder ein Alaninat-Tensid ausgewählt aus Natriumcocoyalaninat, Natriumlauroylalaninat, Natrium-N-Dodecanoyl-I-alaninat und Kombinationen davon, wobei die Gesamtmenge von primärem anionischen Tensid in einem Bereich von 35 Gew.-% bis 90 Gew.-%, auf Basis eines trockenen Artikels, liegt; und
ii. zu 10 Gew.-% bis 65 Gew.-%, vorzugsweise zu 15 Gew.-% bis 55 Gew.-%, mehr bevorzugt zu 23 Gew.-% bis 50 Gew.-% des Tensidsystems, auf Basis eines trockenen Artikels, ein Co-Tensid;
wobei das Tensidsystem weniger als 0,1 % sulfatbasierte Tenside bezogen auf das Gesamtgewicht der faserigen Elemente umfasst;

c. zu 0,5 Gew.-% bis 5 Gew.-%, vorzugsweise zu 0,75 Gew.-% bis 3,7 Gew.-%, mehr bevorzugt zu 1 Gew.-% bis 3 Gew.-%, auf Basis des trockenen Artikels, ein pH-Einstellmittel, wobei das pH-Einstellmittel aus einer monoprotischen organischen Säure besteht; und wobei die monoprotonische organische Säure in den faserigen Elementen dispergiert ist, wobei die monoprotonische organische Säure ausgewählt ist aus der Gruppe, bestehend aus Milchsäure, Essigsäure, Glykolsäure, Glycerinsäure und Kombinationen davon, wobei die Gesamtmenge von pH-Einstellmittel in einem Bereich von 0,5 Gew.-% bis 5 Gew.-%, auf Basis des trockenen Artikels, liegt;
d. wobei die faserigen Elemente weniger als 0,1 % Citronensäure bezogen auf das Gesamtgewicht der faserigen Elemente umfassen;
e. wobei die Vielzahl von faserigen Elementen miteinander verflochten oder anderweitig miteinander verbunden sind, um den faserigen Artikel auszubilden.

**2.** Artikel nach Anspruch 1, wobei die monoprotonische organische Säure Milchsäure umfasst.

3. Artikel nach einem der vorstehenden Ansprüche, wobei die faserigen Elemente homogen sind.

4. Artikel nach einem der vorstehenden Ansprüche, wobei der Artikel ferner ein kationisches Polymer umfasst, das ausgewählt ist aus der Gruppe, bestehend aus Polyquaternium-6, Polyquaternium-10, kationischen Guars und Kombinationen davon.

5. Artikel nach einem der vorstehenden Ansprüche, wobei der Artikel einen Handauflösungswert von weniger als 154 Hüben, vorzugsweise weniger als 12 Hüben, mehr bevorzugt weniger als 10 Hüben gemäß dem hierin offenbarten Handauflösungsprüfverfahren umfasst.

6. Artikel nach einem der vorstehenden Ansprüche, wobei der Artikel ferner eine Beschichtung umfasst, umfassend ein Additiv, das ausgewählt ist aus der Gruppe, bestehend aus Hydrotropen, diprotischen und triprotischen organischen Säuren und Salzen davon, und Kombinationen davon.

7. Artikel nach Anspruch 6, wobei die diprotischen und triprotischen organischen Säuren ausgewählt sind aus der Gruppe, bestehend aus Citronensäure, Oxalsäure, Malonsäure, Tartronsäure, Fumarsäure, Maleinsäure, Äpfelsäure, Weinsäure und Salzen davon und Kombinationen davon.

8. Artikel nach Anspruch 7, wobei die diprotische und triprotische organische Säure Citronensäure und Salze davon umfasst.

9. Artikel nach Anspruch 6, wobei die Hydrotrope ausgewählt sind aus der Gruppe, bestehend aus Natriumxylolsulfonat, Harnstoff, Natriumtoluolsulfonat und Kombinationen davon.

10. Artikel nach einem der vorstehenden Ansprüche, wobei der Artikel ein G' von 5 Pa bis 150 Pa bei 25 °C umfasst, vorzugsweise von 10 Pa bis 100 Pa bei 25 °C, mehr bevorzugt von 12 Pa bis 75 Pa bei 25 °C, gemäß dem hierin offenbarten Rheologieprüfverfahren, wenn der Artikel mit 7 ml Leitungswasser pro 2,5 Gramm Artikel hydratisiert wird.

11. Verwendung des Artikels nach einem der vorstehenden Ansprüche zum Bereitstellen einer Shampoozusammensetzung, die glatt und cremig ist, wenn der Artikel hydratisiert ist.

## Revendications

1. Article de shampooing fibreux solide pouvant se dissoudre comprenant une pluralité d'éléments fibreux comprenant :

a. de 1 % à 50 %, de préférence de 20 % à 70 %, plus préférablement de 35 % à 60 %, en poids sur une base d'article sec, d'un structurant polymère, dans lequel le structurant polymère est choisi parmi carboxyméthylcellulose, amidon, alcool polyvinylique, ou combinaisons de ceux-ci, dans lequel la quantité totale de structurant polymère va de 1 % à 50 % en poids sur une base d'article sec ;
b. de 20 % à 70 %, de préférence de 40 % à 70 %, plus préférablement de 45 % à 65 %, en poids sur une base d'article sec, d'un système tensioactif comprenant :

i. de 35 % à 90 %, de préférence de 45 % à 85 %, plus préférablement de 50 % à 80 % en poids du système tensioactif sur une base d'article sec, d'un agent tensioactif anionique primaire, dans lequel l'agent tensioactif anionique primaire comprend un agent tensioactif glutamate choisi dans le groupe constitué de cocoyl-glutamate sodique, cocoyl-glutamate disodique, cocoyl-glutamate potassique, cocoyl-glutamate dipotassique, cocoyl-glutamate d'ammonium, cocoyl-glutamate de diammonium, cocoyl-glutamate de TEA, ou des combinaisons de ceux-ci ou un agent tensioactif alaninate choisi parmi cocoyl-alaninate sodique, lauroyl-alaninate sodique, N-dodécanoyl-1-alaninate sodique, et combinaisons de ceux-ci, dans lequel la quantité totale d'agent tensioactif anionique primaire va de 35 % à 90 % en poids sur une base d'article sec ; et
ii. de 10 % à 65 %, de préférence de 15 % à 55 %, plus préférablement de 23 % à 50 % en poids du système tensioactif sur une base d'article sec, d'un co-tensioactif ;
dans lequel le système tensioactif comprend moins de 0,1 % d'agents tensioactifs à base de sulfate en poids total des éléments fibreux ;

c. de 0,5 % à 5 %, de préférence de 0,75 % à 3,7 %, plus préférablement de 1 % à 3 %, en poids sur une base

d'article sec, d'un ajusteur de pH, dans lequel l'ajusteur de pH est constitué d'un acide organique monoprotique ; et dans lequel l'acide organique monoprotique est dispersé sur l'ensemble des éléments fibreux, dans lequel l'acide organique monoprotique est choisi dans le groupe constitué d'acide lactique, acide acétique, acide glycolique, acide glycérique et combinaisons de ceux-ci, dans lequel la quantité totale d'ajusteur de pH va de 0,5 % à 5 % en poids sur une base d'article sec ;
d. dans lequel les éléments fibreux comprennent moins de 0,1 % d'acide citrique en poids total des éléments fibreux ;
e. dans lequel la pluralité d'éléments fibreux sont entremêlés ou autrement associés les uns aux autres pour former l'article fibreux.

**2.** Article selon la revendication 1, dans lequel l'acide organique monoprotique comprend de l'acide lactique.

**3.** Article selon de quelconques revendications précédentes, dans lequel les éléments fibreux sont homogènes.

**4.** Article selon de quelconques revendications précédentes, dans lequel l'article comprend en outre un polymère cationique choisi dans le groupe constitué de polyquaternium-6, polyquaternium-10, gommes de guar cationiques et des combinaisons de ceux-ci.

**5.** Article selon de quelconques revendications précédentes, dans lequel l'article comprend une valeur de dissolution à la main inférieure à 154 passages, de préférence inférieure à 12 passages, plus préférablement inférieure à 10 passages selon le procédé de test de dissolution à la main tel que décrit ici.

**6.** Article selon de quelconques revendications précédentes, dans lequel l'article comprend en outre un revêtement comprenant un additif choisi dans le groupe constitué d'hydrotropes, acides organiques diprotiques et triprotiques et sels de ceux-ci, et combinaisons de ceux-ci.

**7.** Article selon la revendication 6, dans lequel les acides organiques diprotiques et triprotiques sont choisis dans le groupe constitué d'acide citrique, acide oxalique, acide malonique, acide tartronique, acide fumarique, acide maléique, acide malique, acide tartrique, et sels de ceux-ci, et combinaisons de ceux-ci.

**8.** Article selon la revendication 7, dans lequel l'acide organique diprotique et triprotique comprend de l'acide citrique et des sels de celui-ci.

**9.** Article selon la revendication 6, dans lequel les hydrotropes sont choisis dans le groupe constitué de xylène-sulfonate de sodium, urée, toluènesulfonate de sodium, et combinaisons de ceux-ci.

**10.** Article selon de quelconques revendications précédentes, dans lequel l'article comprend un G' allant de 5 Pa à 150 Pa à 25 °C, de préférence de 10 Pa à 100 Pa à 25 °C, plus préférablement de 12 Pa à 75 Pa à 25 °C, selon le procédé de test de rhéologie tel que décrit ici, lorsque l'article est hydraté avec 7 mL d'eau de distribution pour 2,5 grammes d'article.

**11.** Utilisation de l'article selon de quelconques revendications précédentes pour la fourniture d'une composition de shampooing qui est lisse et crémeuse lorsque l'article est hydraté.

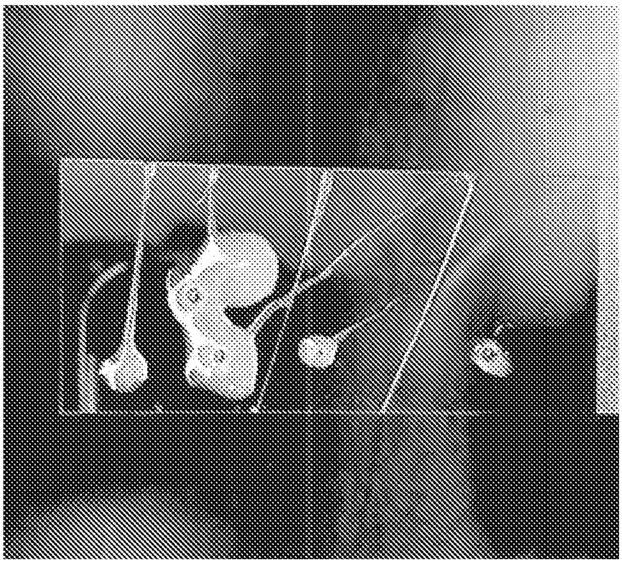
Fig. 1A

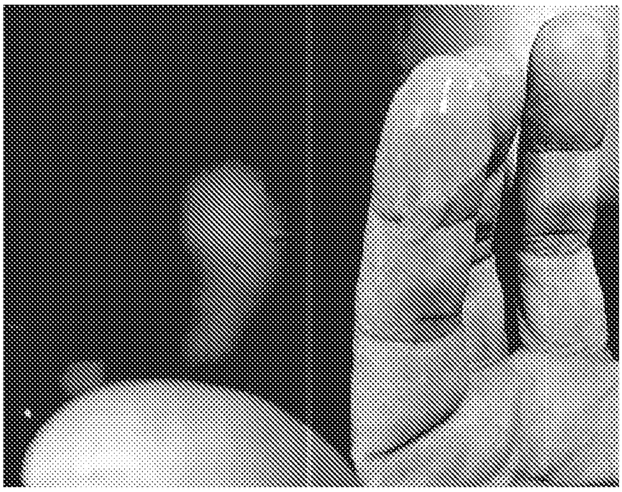
Fig. 1B

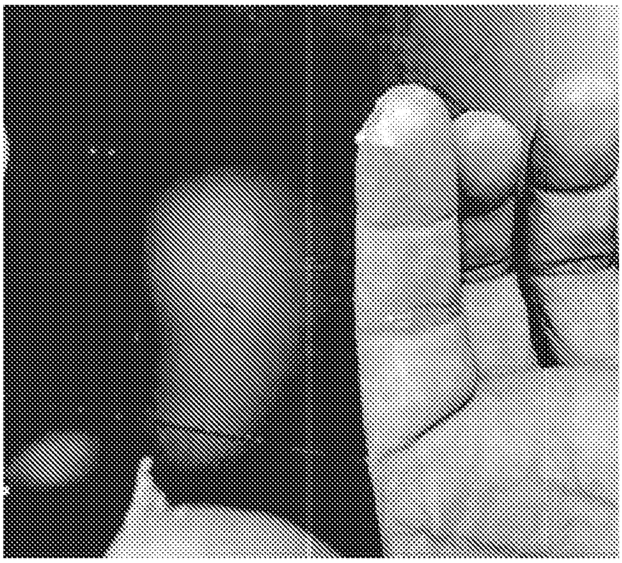
Fig. 1C

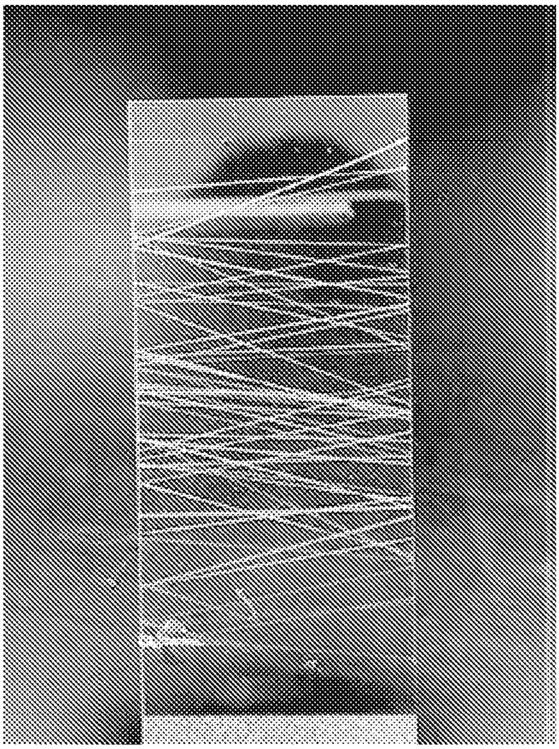

Fig. 2A

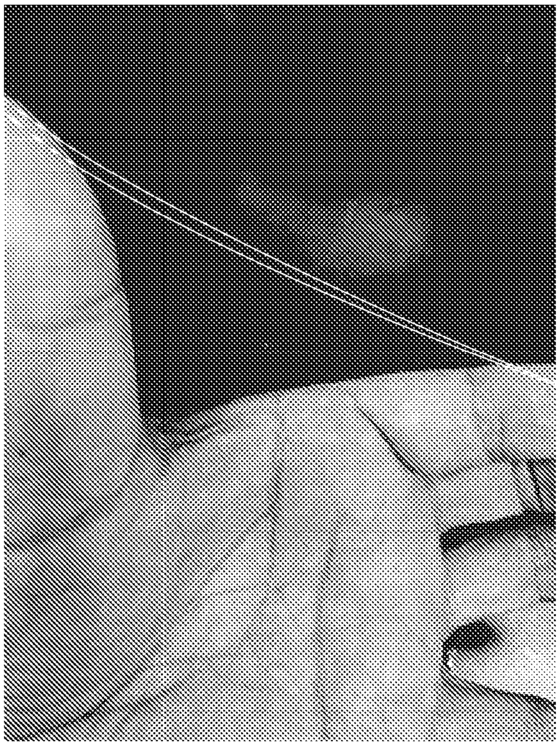

Fig. 2B

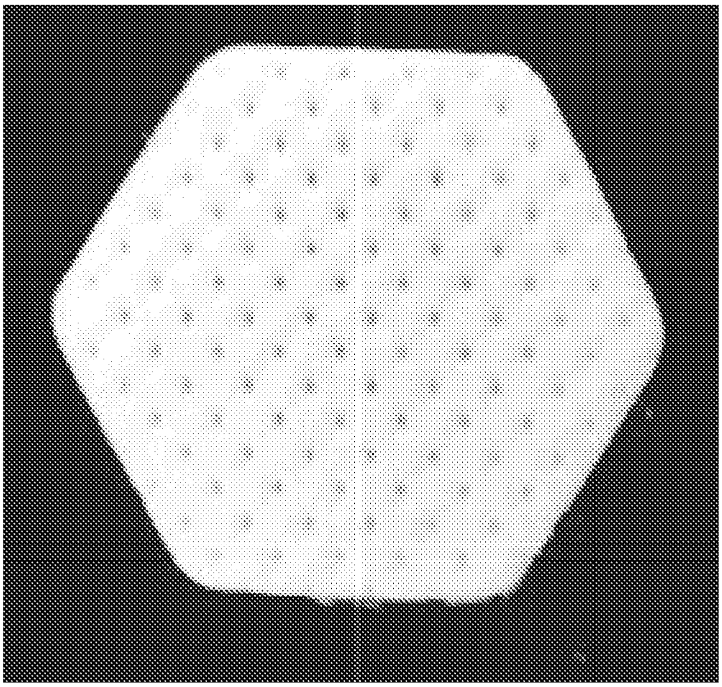

Fig. 3

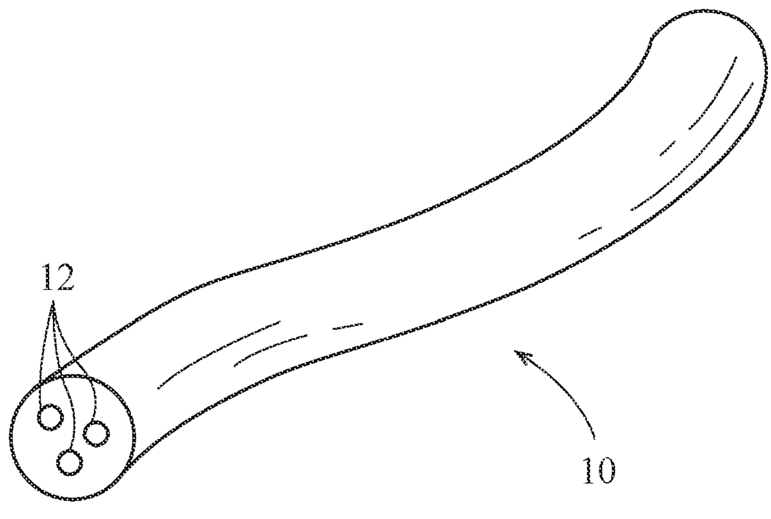


Fig. 4

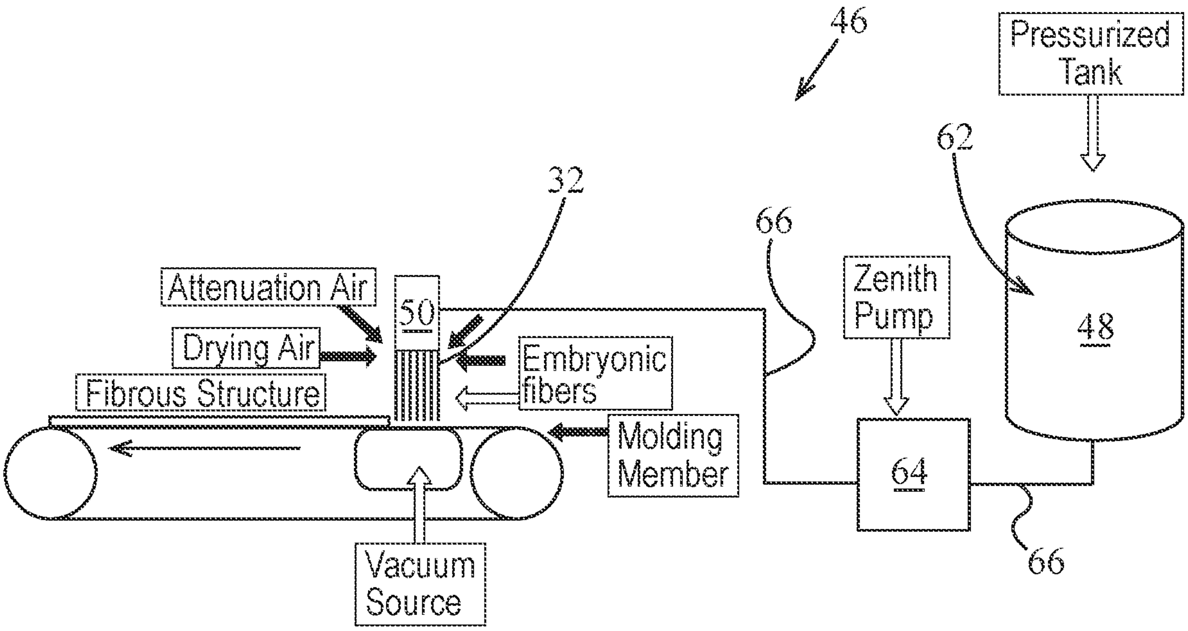
46
Pressurized Tank
62
32
66
Zenith Pump
48
Attenuation Air
50
Drying Air
Embryonic fibers
Fibrous Structure
Molding Member
64
66
Vacuum Source

Fig. 5

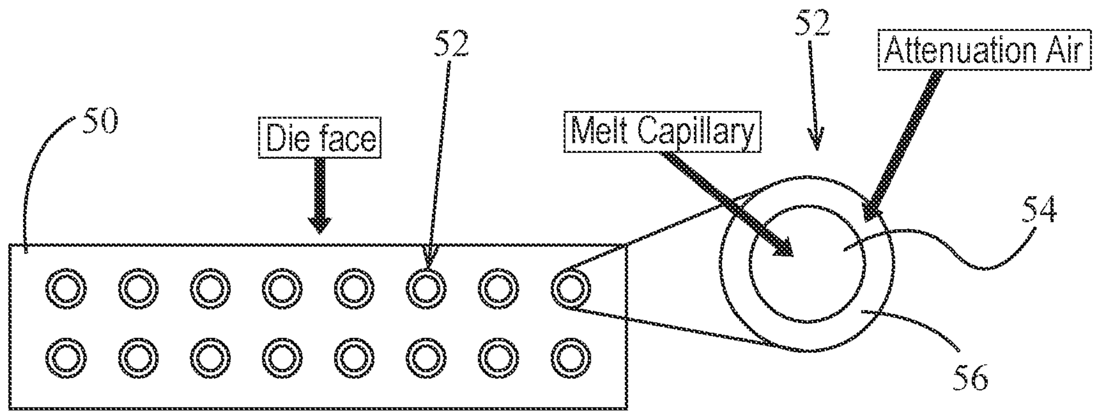


Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018140676 A2
- US 2014329428 A1
- US 8980816 B
- US 9139802 B
- US 20130171421 A
- US 912876
- US 431115
- US 2486921 A
- US 2486922 A
- US 2396278 A
- US 5104646 A
- US 5106609 A
- US 2658072 A
- US 2438091 A
- US 2528378 A
- EP 0283165 B1
- US 20080019935 A
- US 20080242584 A
- US 20060217288 A


### Non-patent literature cited in the description


- **SOLAREK, D. B.** Cationic Starches in Modified Starches: Properties and Uses. CRC Press, Inc., 1986, 113-125
- **QIN-JI PENG** ; **ARTHUR S. PERLIN**. Observation on NMR Spectra of Starches in Dimethyl Sulfoxide, Iodine-Complexing, and Solvating in Water-Dimethyl Sulfoxide. *Carbohydrate Research*, 1987, vol. 160, 57-72
- **J. HOWARD BRADBURY** ; **J. GRANT COLLINS**. An Approach to the Structural Analysis of Oligosaccharides by NMR Spectroscopy. *Carbohydrate Research*, 1979, vol. 71, 15-25
- CTFA Cosmetic Ingredient Handbook. The Cosmetic, Toiletries, and Fragrance Association, Inc., 1992